(19) Europäisches Patentamt European Patent Office Office européen des brevets

(11) **EP 2 736 472 B1**

(12) **EUROPEAN PATENT SPECIFICATION**

(45) Date of publication and mention of the grant of the patent:
**07.09.2016 Bulletin 2016/36**

(21) Application number: **12819192.1**

(22) Date of filing: **27.07.2012**

(51) Int Cl.:
*A61F 13/49* (2006.01)     *A61F 13/15* (2006.01)
*A61F 13/53* (2006.01)

(86) International application number:
**PCT/JP2012/004812**

(87) International publication number:
**WO 2013/018346 (07.02.2013 Gazette 2013/06)**

(54) **BODILY FLUID ABSORBENT MATRIX AND METHOD FOR MANUFACTURING THE SAME**

KÖRPERFLÜSSIGKEITSABSORBIERENDE MATRIX UND VERFAHREN ZU IHRER HERSTELLUNG

MATRICE ABSORBANT LES FLUIDES CORPORELS ET PROCÉDÉ DE FABRICATION DE CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2011 JP 2011167543**

(43) Date of publication of application:
**04.06.2014 Bulletin 2014/23**

(73) Proprietor: **Unicharm Corporation**
**Ehime 799-0111 (JP)**

(72) Inventors:
• **DETANI, Kou**
**Kanonji-shi**
**Kagawa 769-1602 (JP)**
• **GODA, Hiroki**
**Kanonji-shi**
**Kagawa 769-1602 (JP)**

(74) Representative: **Dolleymores**
**9 Rickmansworth Road**
**Watford, Hertfordshire WD18 0JU (GB)**

(56) References cited:
| | |
|---|---|
| **EP-A2- 1 253 231** | **WO-A1-2011/122055** |
| **WO-A2-02/056812** | **DE-A1-102008 007 804** |
| **JP-A- 1 195 856** | **JP-A- 2 004 362** |
| **JP-A- 3 173 562** | **JP-A- 2004 275 352** |
| **JP-A- 2011 115 547** | **JP-A- 2011 115 547** |
| **JP-A- 2011 212 290** | **US-A- 4 636 209** |
| **US-A1- 2004 122 394** | |

## Description

### Technical Field

The present disclosure relates to bodily fluid absorbent matrixes suitable for use in bodily fluid absorbent wearing articles such as disposable diapers and sanitary napkins and to methods for manufacturing the absorbent matrixes.

### Background

Absorbent structures used for bodily fluid absorbent wearing articles are known. Bodily fluid absorbent cores (matrixes) included in such absorbent structures are also known. Concerning the absorbent structures and the matrixes, it is known that these absorbent structures and cores may be provided with density gradients in the thickness direction to accelerate the movement of bodily fluids in the thickness direction and thereby to control the accumulation of bodily fluids in the thickness direction.

For example, in an absorbent structure exemplified in JP H03-173562 A1 (PTL 1), a low density fibrous layer formed of fluff wood pulp is stacked on a pulp layer compressed to have a high density wherein the low density fibrous layer faces the article wearer's skin. The low density fibrous layer is needed to ensure a high water absorbability and a high water holding property and the high density fibrous layer functions as a bodily fluid dispersant layer.

JP H10-309298 A1 (PTL 2) discloses a disposable diaper as one example of an absorbent article, and an absorbent structure in this diaper includes a first absorbent region lying on the side of a liquid-permeable topsheet and having a density in the range of 0.03 to 0.20g/cm$^3$, a third absorbent region lying on the side of a liquid-impermeable backsheet and having a density in the range of 0.03 to 0.20g/cm$^3$ and a second absorbent region lying between the first and third absorbent regions and having a density in the range of 0.1 to 0.40g/cm$^3$. In this diaper, urine discharged on the topsheet is spot-absorbed by the first absorbent region, dispersed in a boundary face between the first absorbent layer and the second absorbent layer and retained in the second absorbent layer and the third absorbent layer.

Disposable toilet-training pants disclosed in JP H05-59602 A1 (PTL 3) include a liquid-permeable topsheet, a liquid-impermeable backsheet and a liquid-absorbent core interposed between these top- and backsheets, wherein the topsheet is provided in a central zone thereof with a moisture sensor means. The moisture sensor means includes a part of the topsheet and moisture retention sheet stacked on the upper surface of the topsheet. The moisture retention sheet is a nonwoven fabric including a high density upper layer portion and a low density lower layer portion wherein a density of the topsheet is higher than a density of the lower layer portion. In the toilet-training pants, bodily fluids stay in the upper layer portion of the moisture retention sheet and come in contact with baby's skin and make the baby uncomfortable.

WO 2011/122055 A1 provides a bodily fluid-absorbent structure having a core material formed of an aggregation of fluff wood pulp fibers.

WO 02/056812 A1 discloses a thin, high capacity multi-layer absorbent core. An upper layer displays intake properties and a lower layer displays distribution properties.

US 2004/122394 A1 relates to an absorbent core including a stabilised first absorbent layer and a second absorbent layer that contains a superabsorbent and absorbent fibers treated with a non-fugitive densification agent.

JP2011 115547A discloses a method for thinning the thickness of an assembly of an absorbent material.

### Citation List

#### Patent Literature

PTL 1: JP H03-173562 A1

PTL 2: JP H10-309298 A1

PTL 3: JP HOS-59602 A1

### Summary of Invention

#### Technical Problem

In a bodily fluid absorbent core (matrix) used for the absorbent structure of a bodily fluid absorbent wearing article, a mass or aggregate of hydrophilic fibers contained therein may be provided with a density gradient to move the bodily fluids discharged thereon from the low density region to the high density region. For this reason, a matrix including a low density region lying close to the wearer's skin and a high density region lying remote from the wearer's skin ensures the bodily fluids absorbed by the matrix move away from the low density region lying close to the wearer's skin to the high density region and thereby restricts a wet feeling due to the bodily fluids.

**Solution to Problem**

The present invention includes a first aspect and a second aspect.

The first aspect of this invention relates to a bodily fluid absorbent matrix including an aggregate of bodily fluid absorbent material interposed between a liquid-permeable first sheet lying on a skin-facing side of a bodily fluid absorbent wearing article and a liquid-permeable or liquid-impermeable second sheet lying on a garment-facing side opposite to the skin-facing side.

The first aspect of this invention resides in that: the absorbent matrix includes hydrophilic fibers serving as the bodily fluid absorbent material; the absorbent matrix has a density gradient so as to increase from the first sheet toward the second sheet in a thickness direction of the absorbent matrix; a planar orientation index of the hydrophilic fibers increases in the thickness direction; the absorbent matrix has an upper layer portion and a lower layer portion overlapping each other in the thickness direction so that the upper layer portion faces the first sheet and the lower layer portion faces the second sheet; the density of the upper layer portion is lower than that of the lower layer portion; and the planar orientation index in the upper layer portion is less than 1.30 and the planar orientation index in the lower layer portion is at least 0.3 larger than the planar orientation index in the upper layer portion.

The second aspect of this invention relates to a method for manufacturing a bodily fluid absorbent matrix including an aggregate of bodily fluid absorbent material interposed between a liquid-permeable first sheet lying on a skin-facing side of a bodily fluid absorbent wearing article and a liquid-permeable or liquid-impermeable second sheet lying on a garment-facing side opposite to the skin-facing side.

The second aspect of this invention resides in that the method of the manufacturing the bodily fluid absorbent matrix includes at least the steps as follows:

(1) a step of providing the absorbent matrix with: an upper layer portion and a lower layer portion which are stacked on each other in the thickness direction and have the same thickness; and the aggregate including hydrophilic fibers as the bodily fluid absorbent material, and a plurality of first aggregates each formed of a portion of the bodily fluid absorbent material intended to form the lower layer portion are arranged intermittently in the machine direction and transported in the machine direction;

(2) a step of interposing the respective first aggregates between a pair of air-permeable first support means running in the machine direction between a first nozzle array for high pressure steam jets and a first suction port for suction of steam so that the respective first aggregates are subjected to high pressure steam jets from the first nozzle array under the suction effect of the first suction port to obtain second aggregates corresponding to the first aggregates compressed in the thickness direction thereof;

(3) a step of stacking a plurality of third aggregates of bodily fluid absorbent material intended to form the upper layer portions on the respective second aggregates to obtain fourth aggregates; and

(4) a step of interposing the fourth aggregates between a pair of air-permeable second support means running in the machine direction between a second nozzle array for high pressure stream jets and a second suction port for suction of steam facing each other so that the third aggregates in the fourth aggregates are subjected to high pressure steam jets from the second nozzle array under the suction effect from the second suction port to compress the fourth aggregates and thereby to obtain fifth aggregates from the fourth aggregates.

The term "thickness" as used in this invention refers to a thickness measured with use of a dial thickness gauge. A dial thickness gauge including a stationary side probe having a diameter of 50mm and a movable side probe having a diameter of 50mm may be used, wherein the movable side probe is subjected to a measuring pressure of 0.025N/100mm$^2$-. One example of such a dial thickness gauges is Dial Thickness Gauge J-B type manufactured by OZAKI MFG CO., LTD. in Japan.

The term "fiber length" as used herein with respect to fibers which are pulp fibers such as fluff wood pulp fibers refers to the length-weighted average fiber length as measured with the use of a Kajaani Fiber Lab FS300 manufactured by Metso Automation Co., Ltd. For fibers other than pulp fibers, "fiber length" refers to the averaged fiber length as measured by the C method prescribed by Item c of Paragraph 8-4-1 of Japanese Industrial Standards (JIS) L 1015.

As used herein the term "planar orientation index" refers to a value obtained on the basis of an X-ray CT cross-section photo of an absorbent matrix placed on a plane surface. For the absorbent matrix, X-axis (longitudinal axis), Y-axis (height axis) and Z-axis (transverse axis), being orthogonal to each other, are set and an averaged fiber cross-sectional area per one hydrophilic fiber appearing in an XZ cross-section (cross-section in plane direction), an XY cross-section (cross-section in longitudinal direction) and a YZ cross-section (transverse direction cross-section) are respectively obtained from images of the X-ray CT photo. A ratio of the averaged fiber cross-sectional area per one hydrophilic fiber appearing in the XZ cross-section to the averaged value of the fiber cross-sectional area per one hydrophilic fiber appearing in the XY cross-section and the fiber cross-sectional area per one hydrophilic fiber appearing in the YZ cross-section is defined as the "planar orientation index" of the hydrophilic fibers in the absorbent matrix. Details of the measurement of "planar orientation index" will be described later on.

**Brief Description of Drawings**

[fig.1]Fig. 1 is a partially cutaway perspective view of a bodily fluid absorbent wearing article (sanitary napkin).
[fig.2]Fig. 2 is a sectional view taken along a P-P line in Fig. 1.
[fig.3]Fig. 3 is a diagram illustrating by way of example a process of manufacturing the absorbent matrix.
[fig.4]Fig. 4 is a scale-enlarged partial diagram of Fig. 3.
[fig.5]Fig. 5 (1) to (3) illustrate various cross-sections of a test piece for observation of the fiber orientations.
[fig.6]Fig. 6 is a diagram illustrating by way of example a process of manufacturing the absorbent matrix.
[fig.7]Fig. 7 is a diagram illustrating by way of example a process of manufacturing the absorbent matrix according to a Comparative Example.
[fig.8]Fig. 8 is a diagram illustrating by way of example a process of manufacturing the absorbent matrix according to a Comparative Example.

**Description of Embodiments**

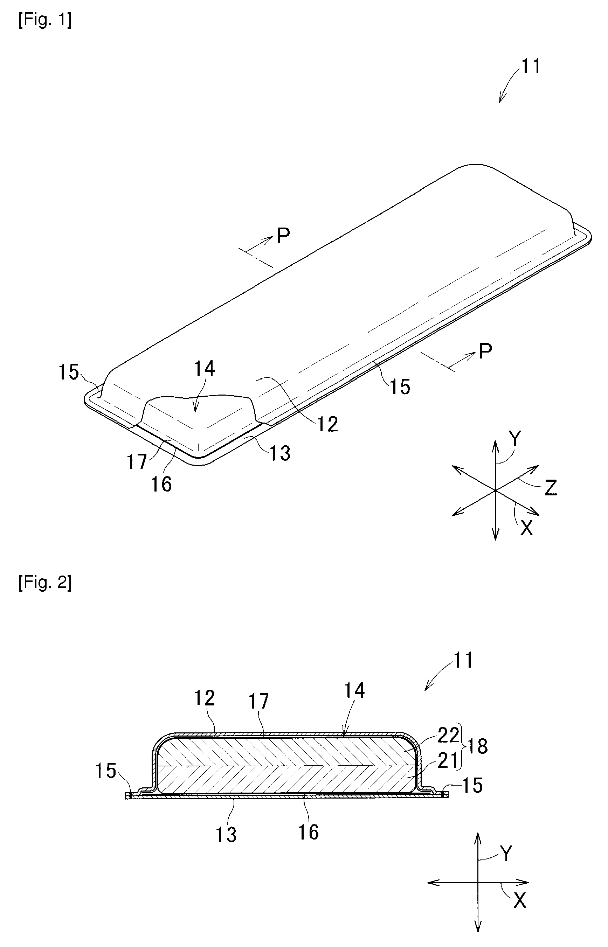

Referring to Figs. 1 and 2, a sanitary napkin 11 includes a liquid-permeable topsheet 12, a liquid-impervious backsheet 13 and an absorbent structure 14 interposed between these two sheets 12, 13. These two sheets 12, 13 extend outboard of a peripheral edge of the absorbent structure 14 and are put flat together along extensions thereof lying outboard of the peripheral edges of the absorbent structure 14 and sealed together along a seal line 15 extending so as to surround the absorbent structure 14. In Fig. 1, a transverse direction, a thickness direction and a longitudinal direction of the napkin, being orthogonal to each other, are indicated by double-headed arrows X, Y and Z.

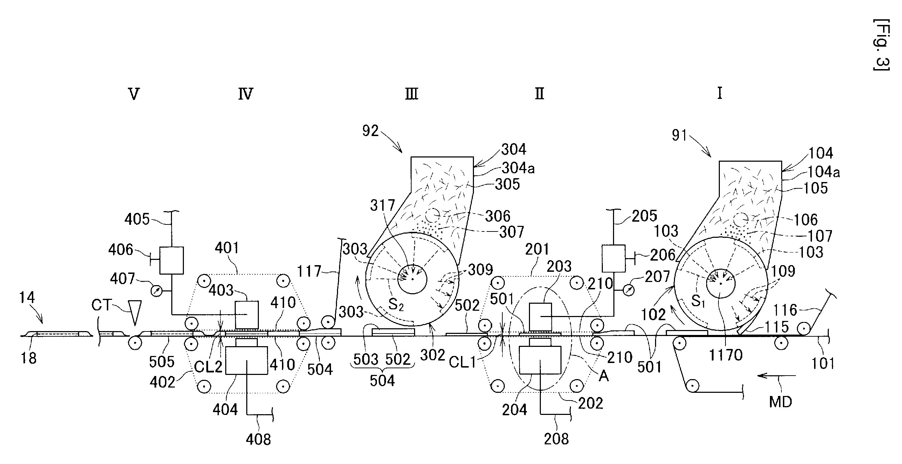

The absorbent structure 14 interposed between the topsheet 12 and the backsheet 13 in Fig. 1 includes a liquid-permeable upper wrapping sheet 17, a liquid-permeable or liquid-impermeable lower wrapping sheet 16, and a bodily fluid absorbent matrix 18 serving as an absorbent core interposed between these wrapping sheets 16, 17 wherein the wrapping sheets 16, 17 extend outboard beyond a peripheral edge of the absorbent matrix 18 and are put flat together along the respective extensions beyond the peripheral edge of the absorbent matrix 18. The absorbent matrix 18 includes a lower layer portion 21 and an upper layer portion 22 which are stacked on each other in the thickness direction and have substantially the same thickness. The upper layer portion 22 is adapted to absorb the bodily fluids such as menstrual blood having permeated the topsheet 12 and the upper wrapping sheet 17 and then to transfer the absorbed bodily fluids downward in the direction Y, i.e., to the lower layer portion 21. The lower layer portion 21 is adapted to absorb the bodily fluids coming from the upper layer portion 22 and then to disperse the absorbed bodily fluids in the transverse direction X and in the longitudinal direction Z. Details of the structure of such an absorbent matrix 18 and a method for manufacturing such an absorbent matrix 18 will be described with reference primarily to Fig. 3.

In such a sanitary napkin 11, the topsheet 12 may be formed of commonly used materials in the relevant technical field. For example, a nonwoven fabric made of thermoplastic synthetic fibers such as a spun bonded nonwoven fabric, a melt bonded nonwoven fabric, a melt blown nonwoven fabric, or an SMS nonwoven fabric composed of a spun bonded nonwoven fabric, a melt blown nonwoven fabric and a spun bonded nonwoven fabric laminated together in this order, or a perforated plastic film may be used. When a nonwoven fabric is used, the mass per unit area thereof is preferably in a range of from 10 to 25g/m$^2$. When a perforated plastic film is used, a plastic film having a thickness in a range of from 0.005 to 0.03mm may be subjected to a perforating treatment of known art. Both the nonwoven fabric and the plastic film are preferably modified to become hydrophilic.

The backsheet 13 also may be formed of materials widely used in the relevant technical field. For example, a liquid-impermeable plastic film or an air-permeable but liquid-impermeable plastic film having a thickness in a range of from 0.01 to 0.03mm may be used. Alternatively, a nonwoven fabric made of thermoplastic synthetic fibers may be laminated on the outer surface of the plastic film to provide the backsheet with a fabric-like texture and/or laminated on a relatively thin plastic film to provide the backsheet with a flexible and comfortable texture.

The upper wrapping sheet 17 may be formed of tissue paper or of a nonwoven fabric made of thermoplastic synthetic fibers, and modified to become hydrophilic. In the latter case, the mass per unit area of such a nonwoven fabric is preferably in the range of from 5 to 20g/m$^2$.

As the lower wrapping sheet 16, it is possible to use tissue paper or nonwoven fabric the same as those used for the upper wrapping sheet. In this regard, however, when the liquid-impermeable lower wrapping sheet 16 is desired, a liquid-impermeable plastic film or a substantially liquid-impermeable nonwoven fabric may be used.

In the absorbent matrix 18, the upper layer portion 22 and the lower layer portion 21 are formed of aggregates of bodily fluid absorbent materials, respectively. The aggregates include hydrophilic fibers in an amount of from 100 to 25% by mass (corresponding to 75% by mass to be described later on) and superabsorbent polymer particles in an amount of from 0 to 75% by mass, wherein, as the hydrophilic fibers, for example, fluff wood pulp, rayon fibers, acetate fibers or thermoplastic synthetic fibers modified to become hydrophilic may be used. The fiber length of the hydrophilic fibers is preferably 15mm or less, more preferably 10mm or less and even more preferably 5mm or less. The fineness of the rayon fibers, the acetate fibers or the thermoplastic synthetic fibers modified to become hydrophilic is preferably 8dtex or less and more preferably 4dtex or less.

As the superabsorbent polymer particles, those commonly used in the relevant technical field may be selected. For example, particles formed of polyacrylic acid, polyacrylate, starch-acrylonitrile copolymer, polyvinyl alcohol, polyvinyl ether, poly-aciylamide, carboxymethylcellulose or natural polysaccharide may be used.

The upper layer portion 22 is the portion in which the mass per unit area is preferably in the range of from 50 to 250g/m$^2$ and the specific volume is preferably in the range from 5 to 25cm$^3$/g. The lower layer portion 21 is the portion in which the mass per unit area is preferably in the range of from 100 to 450g/m$^2$, the specific volume is preferably in the range of from 2.5 to 13cm$^3$/g, the mass per unit area is preferably equal to or larger than that of the upper layer portion 22 and the specific volume is preferably equal to or less than that of the upper layer portion 22. In an absorbent matrix 18 including such a lower layer portion 21 and upper layer portion 22, the density changes to increase from the upper layer portion 22 toward the lower layer portion 21. In other words, the specific volume changes to decrease from the upper layer portion 22 toward the lower layer portion 21.

Referring to Fig. 3 as a diagram illustrating by way of example a series of the steps of a manufacturing process of the absorbent matrix 18, in a first step I, a first carrier web 116 destined for the lower wrapping sheets 16 is placed on a mesh conveyor belt 101 running in a machine direction MD so as to pass below a first molding drum 102. The first step I involves the first molding drum 102 adapted to rotate clockwise as viewed in Fig. 3 and a first material feeding station 104. The first molding drum 102 has formed on its peripheral surface a plurality of first recesses 103 used to mold the portions corresponding to the lower layer portions 21 of the respective matrixes 18, circumferentially arranged. These recesses 103 are indicated by chain lines. The first recesses 103 enter the first material feeding station 104 when the first molding drum 102 rotates together with the first recesses 103 and approaches the 12 o'clock position. The first material feeding station 104 includes a conduit 104a within which first hydrophilic fibers 105 adapted to form the lower layer portions 21 of the respective matrixes 18 are fed from above. A first polymer feeder 106 indicated within the conduit 104a by a chain line is used when it is preferable that the lower layer portions 21 of the respective matrixes 18 contain superabsorbent polymer particles. When the first superabsorbent polymer particles 107 are used, the particles are fed from the first polymer feeder 106 to the first recesses 103 to mix with the first hydrophilic fibers 105. The first recesses 103 are put in communication with a first suction unit 1170 located inside the first molding drum 102 when these first recesses 103 are supplied with a mixture of the first hydrophilic fibers 105 and the first superabsorbent polymer particles 107 so that the mixture may be sucked toward the first recesses 103 and deposited thereinto. After leaving the first material feeding station 104, the deposited mixture is now subjected to the effect of release of air 109 from inside the first molding drum 102 and released from the first recesses 103 and run in the machine direction MD in the form of first aggregates 501.

In this regard, immediately after the first recesses 103 have entered the first material feeding station 104, the first hydrophilic fibers 105 fed into the first recesses 103 are deposited in the first recesses 103 so as to be oriented along the flat bottoms of the first recesses 103 under the suction effect $S_1$ of the first suction unit 1170, in other words, so as to lie down on the bottoms of the first recesses 103. However, the first hydrophilic fibers 105 subsequently fed are less influenced by the suction effect $S_1$ due to the presence of the already deposited first hydrophilic fibers and are apt to orient in a depth direction of the respective first recesses, i.e., in a radial direction of the first molding drum 102 rather than lying down on the bottoms of the first recesses 103.

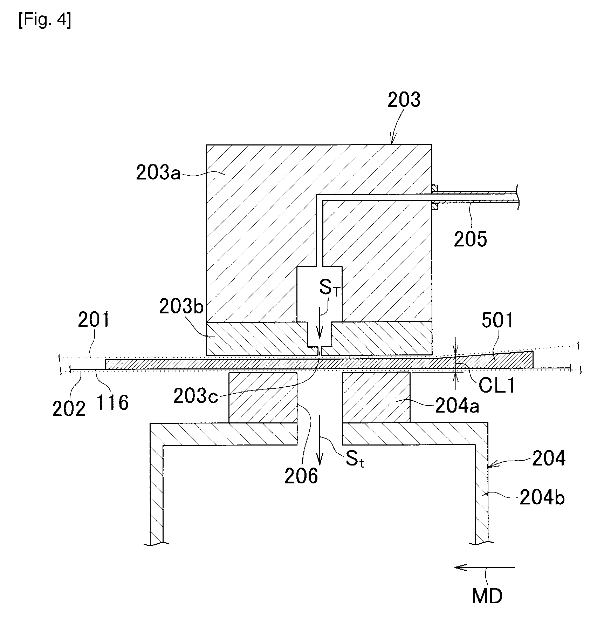

Now the first aggregates 501 proceed to a second step II. The second step II involves a first endless belt 201 and a second endless belt 202 both formed of air-permeable mesh belts and having segments 210 running in parallel to each other in the machine direction MD. Immediately above and below the respective segments 210 of the first and second endless belts 201, 202, respectively, there are provided a first block 203 for high-pressure steam jets and a first box 204 for steam suction, located so as to be opposed to each other. A plumbing unit 205 to supply high-pressure steam is connected to the first block 203 and this plumbing unit is provided with a pressure control valve 206 and a pressure gauge 207. The first block 203 and the first box 204 are constructed so that a spacing distance CL1 (See Fig. 4) between them can be adjusted. The first aggregates 501 interposed between the first and second endless belts 201, 202 and running between the first block 203 and the first box 204 having the spacing distance CL1 appropriately adjusted are subjected to the high-pressure steam jets from the first block 203 (See Fig. 4) and this steam is sucked by the first box 204. The first aggregates 501 are compressed by the high-pressure steam jets and have their thickness reduced and at the same time the first hydrophilic fibers 105 in these first aggregates 501 are redistributed. In this way, the first aggregates 501 are changed to second aggregates 502 which leave the first and second endless belts 201, 202 and proceed to a third step III.

The third step III of Fig. 3 involves a second molding drum 302 rotating clockwise and a second material feeding station 304. The second molding drum 302 is formed on its peripheral surface with a plurality of second recesses 303 used to mold the portions corresponding to the upper layer portions 22 of the respective absorbent matrixes 18 circumferentially arranged. These recesses 303 are indicated by chain lines in Fig. 3. The second recesses 303 enter the second material feeding station 304 when the second molding drum 302 approaches together with the second recesses 303 the 12 o'clock position. The second material feeding station 304 includes a conduit 304a within which second hydrophilic fibers 305 adapted to form the hydrophilic fibers in the upper layer portions 22 of the respective absorbent matrixes 18 are fed from above. A second polymer feeder 306 indicated within the conduit 304a by a chain line is used only when the upper layer portions 22 of the respective matrixes 18 contain superabsorbent polymer particles. In such a case, second superabsorbent polymer particles 307 are fed from the second polymer feeder 306 to the second recesses 303, mixing with the second hydrophilic fibers 305. The second recesses 303 are put in communication with a second suction unit 317 located inside the second molding drum 302 when these second recesses 303 are supplied with the second hydrophilic fibers 305 and the second superabsorbent polymer particles 307 so that the second hydrophilic fibers 305 and the second superabsorbent polymer particles 307 may be sucked toward the second recesses 303 and deposited thereinto. The second hydrophilic fibers 305 having been fed to the second recesses 303 and deposited therein are released from the second recesses 303 under the effect of a release of air 309 and changed to third aggregates 503 of the bodily fluid absorbent material and thereupon stacked on the second aggregates 502, which have already proceeded in the third step III to form fourth aggregates 504 in the form of a layered body thereof. In this regard, in the third step III; the running velocity of the carrier web 116 and the peripheral velocity of the second molding drum 302 are adjusted so that the third aggregates 503 released from the second recesses 303 may be stacked on the second aggregates 502.

In a fourth step IV, a cover web 117 destined for the upper wrapping sheet 17 in Fig. 2 is fed from above as viewed in Fig. 3 to the fourth aggregates 504. In consequence, the fourth aggregates 504 running in the machine direction MD are interposed between the carrier web 116 and the cover web 117. In the fourth step IV, an air-permeable third endless belt 401 and an air-permeable fourth endless belt 402 respectively include segments 410 adapted to run in parallel to each other in the machine direction MD. Immediately above and below these segments 410 of the third and fourth endless belts 401, 402, respectively, a second block 403 for high-pressure steam jets and a second box 404 for steam suction are located so as to be opposed to each other. A plumbing unit 405 to supply high-pressure steam is connected to the second block 403 and this plumbing unit 405 is provided with a pressure control valve 406 and a pressure gauge 407. The second box 404 includes a plumbing unit 408 connected between the second box 404 and a suction source for exhaust and drainage. The second block 403 and the second box 404 are constructed so that a spacing distance CL2 in the vertical direction as viewed in Fig. 3 between them can be adjusted. In the fourth step IV, the third and fourth endless belts 401, 402 run between the second block 403 and the second box 404 having the spacing distance CL2 adjusted and the fourth aggregates 504 composed of the second and third aggregates 502, 503 are interposed between the third and fourth endless belts 401, 402 and also between the carrier web 116 and the cover web 117. In the course of running in this manner, the third aggregates 503 are subjected to high-pressure steam jets (not shown) from the second block 403 and the steam is sucked by the second box 404. The fourth aggregates 504 are compressed by the high-pressure steam jets and have their thickness reduced and at the same time the first hydrophilic fibers 105 and the second hydrophilic fibers 305 in these fourth aggregates 504 are redistributed. In this way, the fourth aggregates 504 are changed to fifth aggregates 505. The carrier web 116 and the cover web 117 are put flat together along peripheral edges of the respective fifth aggregates 505 and closely attached to each other under the effect of the high-pressure stream jets.

In a fifth step V, the carrier web 116 and the cover web 117 are cut by a cutter CT between each pair of the fifth aggregates 505 being adjacent in the machine direction MD to obtain individual absorbent structures 14 composed of the fifth aggregate 505, the carrier web 116 and the cover web 117. The fifth aggregate 505 corresponds to the absorbent matrix 18 in the absorbent structure 14, the carrier web 116 corresponds to the lower wrapping sheet 16 in the absorbent structure 14 and the cut cover web 117 corresponds to the upper wrapping sheet 17 in the absorbent structure 14.

Fig. 4 is a scale-enlarged diagram of a portion A surrounded by an imaginary line in Fig. 3 and illustrates inner structures of the first block 203 and the first box 204, respectively. These inner structures are adopted also for the second block 403 and the second box 404 in the fourth step IV. The first block 203 extends in a cross direction which is orthogonal to the machine direction MD and includes a steam manifold section 203a and a nozzle plate section 203b wherein a plumbing unit 205 used to supply the high-pressure steam $S_T$ is connected to the steam manifold section 203a. The nozzle plate section 203b is formed with a series of first nozzles 203c arranged in the cross direction. The first endless belt 201 slidably runs along the nozzle plate section 203b and therefore the nozzle plate section 203b has a smooth lower surface. The first box 204 includes a slidably supporting plate section 204a and a box body 204b. The slidably supporting plate section 204a is the section along which the second endless belt 202 slidably runs and therefore has a smooth surface. The box body 204b is in communication with a suction source (not shown). The slidably supporting plate section 204a and the box body 204b are formed with a first suction port 206 in a region facing the first nozzles 203c. The slidably supporting plate section 204a is a section which can be replaced by a pair of rolls spaced from each other in the machine direction MD and rotating in the machine direction MD.

Between the nozzle plate section 203b and the slidably supporting plate section 204a, the first and second endless belts 201, 202 run in the machine direction MD and the carrier web 116 and the first aggregates 501 placed on the carrier web 116 are interposed between the first and second endless belts 201, 202. The spacing distance CL1 between the first block 203 and the first box 204 corresponds to a clearance between the nozzle plate section 203b and the slidably supporting plate section 204a and the CL1 is adjusted so that the second aggregates 502 having a required thickness can be obtained from the first aggregates 501 under the high-pressure steam jets $S_t$. In one example of the first block 203, each of the first nozzles 203c arranged in the cross direction CD has a orifice diameter in a range of from 0.1 to 2mm. These nozzles 203c are arranged at a pitch in a range of from 0.5 to 5mm so that the array of these nozzles extends beyond the first aggregates 501 at opposite sides of the array in the cross direction. The high-pressure steam jets $S_t$ ejected from the first block 203 have steam pressure in a range of from 0.1 to 2MPa and such high-pressure steam jets $S_t$ are ejected to the surface of the first aggregates 501 at a rate in the range of from 0.03 to 1.23kg/m$^2$.

As the second block 403 and the second box 404 in the fourth step IV, those having the same construction as the first block 203 and the first box 204 illustrated in Fig. 4 may be used under the same operating conditions as, or different operating conditions from, those in the second step II. In this regard, the second block 403 includes second nozzles (not shown) replacing the first nozzles 203c and the second box 404 includes a second suction port (not shown) replacing the first suction port 206. The spacing distance CL2 between the second block 403 and the second box 404 is larger than the spacing distance CL1.

In the absorbent matrix 18 illustrated in Fig. 2 which has been obtained by the process illustrated in Fig. 3, there is a possibility that the first hydrophilic fibers 105 and the second hydrophilic fibers 305 might be entangled with each other between the lower layer portion 21 and the upper layer portion 22. However, the strength of any such intertwining is relatively weak and the lower layer portion 22 and the upper layer portion 21 can be easily peeled off from each other merely by pinching them with the fingers. In this way, it is easy to observe characteristics of the lower layer portion 21 and the upper layer portion 22 individually.

In the absorbent matrix 18 facilitating its characteristics to be observed, the specific volume is gradually changed so as to decrease from the upper layer portion 22 to the lower layer portion 21. The specific volumes of the upper and lower layer portions 22, 21 are values obtained from the ratios of the volume to mass of these respective volumes of the upper and lower layer portions 22, 21. The volumes are so-called apparent volumes, and thickness values of the upper layer portion 22 and the lower layer portion 21 needed to obtain the so-called apparent volumes thereof are measured with the use of a probe having a mass per unit area of 2.5g/mm$^2$. In the upper layer portion 22, second hydrophilic fibers 306 tend to extend in the thickness direction Y and such a tendency may be described by the expression that, in the absorbent matrix 18 according to this invention, the second hydrophilic fibers 305 tend to be oriented in the thickness direction Y. In the lower layer portion 21, the first hydrophilic fibers 105 tend to extend in a plane orthogonal to the thickness direction Y and such a tendency may be described by the expression that, in the absorbent matrix 18, the first hydrophilic fibers 105 tend to be oriented in the planar direction. These tendencies of orientation can be quantitatively indicated by the planar orientation index obtained on the basis of a measuring method to be described later on.

In the sanitary napkin 11 of Fig. 1 using such an absorbent matrix 18, when menstrual blood discharged on the topsheet 12 is absorbed and reaches the upper layer portion 22, menstrual blood smoothly moves downward to the lower layer portion 21 in the thickness direction Y and, on the lower layer portion 21, menstrual blood is dispersed in the transverse direction X and in the longitudinal direction Z and scarcely comes into contact with the wearer's skin. In consequence, even when menstrual blood is discharged, any wet feeling due to menstrual blood is insignificant.

(Examples)

(Example 1)

A specific example of the absorbent matrix 18 obtained by following the process illustrated in Fig. 3 will be described hereunder. As the first and second hydrophilic fibers 105, 305 fed from the first and second material feeding stations 104, 304 illustrated in Fig. 3, fluff wood pulp fibers NB-416 manufactured by Weyerhauser Co., Ltd. were used. Feed rates of fluff wood pulp fed from the first material feeding station 104 and the second material feeding station 304 were regulated and thereby the first aggregate 501 having a mass per unit area of about 100g/m$^2$ and the third aggregate 503 having a mass per unit area of about 200g/m$^2$ were obtained. NB-416 fluff wood pulp was opened by a sawmill type opener and used as the first and second hydrophilic fibers 105, 305. The average fiber length of the opened NB-416 was 2.47mm. As the carrier web 116 and the cover web 117, an air-through nonwoven fabric having a mass per unit area of 25g/m$^2$, made of core-in-sheath conjugate fibers composed of polyester as the core and polyethylene as the sheath, having a fiber fineness of 2.2dtex and a fiber length of 51mm, was used. In the second and fourth steps II, IV, the steam nozzles each having an orifice diameter of 0.5mm arranged at a pitch of 2mm were used, the steam pressure was set to 0.7MPa and the steam ejection quantity was set to 1.27kg/m$^2$. For the first and second endless belts 201, 202 as well as the third and fourth endless belts 401, 402, the Plain-Woven Mesh Conveyor Belts made of polyphenylene sulfide, manufactured by Nippon Filcon Co., Ltd. in Japan were used. The respective endless belts 201, 202, 401 and 402 had 34 vertical lines each having a line diameter of 0.37mm/inch and 32 transverse lines each having a line diameter of 0.37mm/inch. In the second step II, the spacing distance CL1 was adjusted to change the first aggregates 501 to the second aggregates 502 having a desired thickness under ejection of the high-pressure steam and, in the third step III, the spacing distance CL2 was adjusted to change the fourth aggregates 504 to the fifth aggregates 505 having a desired thickness under ejection of the high-pressure steam. In this way, the absorbent structure 14 was obtained and the absorbent matrix 18 according to Example 1 included in this absorbent structure 14 was obtained.

The lower layer portion 21 and the upper layer portion 22 of the absorbent matrix 18 having been obtained in this manner were evaluated with respect to respective evaluation items described hereunder and the function and effect of the absorbent matrix were made sure. Evaluation results are listed in TABLE 1. It will be easily understood that the lower layer portion 21 of the absorbent matrix 18 corresponds to the lower layer portion of the fifth aggregate 505 in Fig. 3 and this lower layer portion corresponds to the first aggregate 501. The upper layer portion 22 corresponds to the upper layer portion of the fifth aggregate 505 and this upper layer portion corresponds to the third aggregate 503.

(Example 2)

The absorbent matrix 18 of Example 2 was obtained under conditions similar to those in Example 1 except that, in the process illustrated in Fig. 3, the mass per unit area of the first aggregate 501 and the third aggregate 503 was set to 200g/m$^2$ and the spacing distances CL1, CL2 were set to be larger than those in the Example 1. The absorbent matrix 18 according to Example 2 was evaluated with respect to the same evaluation items as those for the absorbent matrix according to Example 1. Evaluation results are listed in TABLE 1.

Evaluation Item 1: Thickness (unit: mm)

(1) Measurement was conducted with use of the method as described in the above Section "Solution to Problem" and, as the unit, mm was used. As test piece for thickness measurement, the absorbent matrix 18 was obtained by peeling off the upper wrapping sheet 17 and the lower wrapping sheet 16 from the absorbent structure 14 obtained by the step illustrated in Fig. 3. While the test piece is preferably dimensioned to be 100 x 100mm, when a test piece smaller than this is used, the diameter of a dial gauge may be made smaller as may be necessary.
(2) To measure thickness of the lower layer portion 21 and the upper layer portion 22, after the total thickness of the absorbent matrix 18 had been measured, the thickness of the lower layer portion 21, obtained by separating these two portions 21, 22 with the finger tips, was measured. The thickness difference between the absorbent matrix 18 and the lower layer portion 21 was calculated to obtain a thickness value for the upper layer portion 22.

Evaluation Item 2: Mass per unit area (basis mass) (unit: g/m$^2$)

(1) The upper wrapping sheet 17 and the lower wrapping sheet 16 were peeled off from an absorbent structure segment measuring of 100 x 100mm cut out from the absorbent structure 14 to obtain a test piece of the absorbent matrix 18 and the mass of this test piece was obtained with the use of an electronic balance.
(2) The mass per unit area (referred to also as a basis mass) of the absorbent matrix 18 was calculated on the basis of the test piece's mass according to an expression as follows:

$$\text{Mass per unit area (basis mass)}(g/m^2) = \text{mass of the test piece}/0.01$$

(3) The mass per unit area of the lower layer portion 21 was obtained with the use of the electronic balance by weighing the lower layer portion 21 left after peeling off the upper layer portion 22 from the test piece of the absorbent matrix 18.
(4) The mass per unit area difference between the absorbent matrix 18 and the lower layer portion 21 was calculated to obtain a mass per unit area of the upper layer portion 22.

Evaluation Item 3: Planar Orientation Index

(1) The term "planar orientation index" used herein refers to the index obtained by recording the first hydrophilic fibers 105 or the second hydrophilic fibers 305 appearing in cross-sections of the lower layer portion 21 and the upper layer portion 22 of the absorbent matrix 18 used as test pieces for observation with X-ray CT photos and measuring cross-sectional areas of these fibers on the basis of these photos. The indices obtained in this manner allow the orientations of the fibers in the cross-sections of the test pieces to be compared.

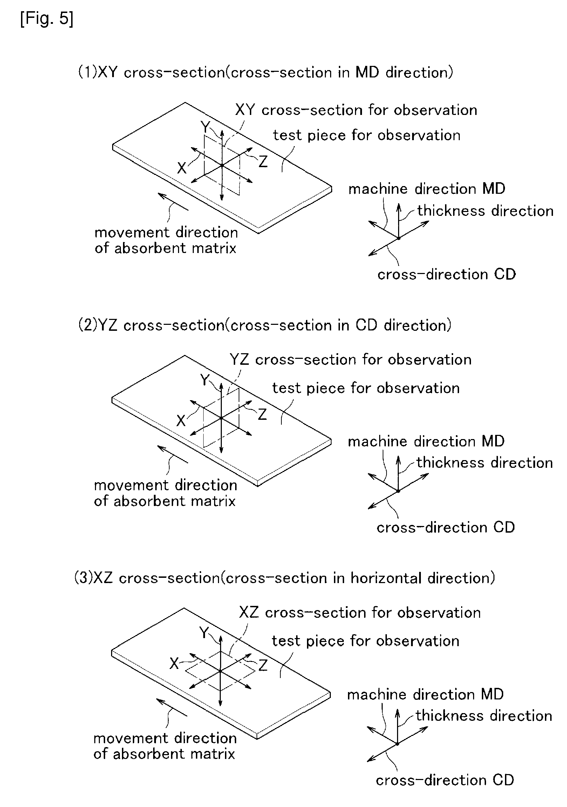

(2) Fig. 5 illustrates cross-sections of the lower layer portion 21 or the upper layer portion 22 used as the test piece for observation to be recorded with X-ray CT photos. Fig. 5 (1) illustrates an XY cross-section of the test piece for observation(cross-section taken in parallel to the machine direction MD) to be described in more detail later. Fig. 5 (2) illustrates a YZ cross-section (cross-section taken in parallel to the cross direction CD) to be described in more detail later and Fig. 5(3) illustrates an XZ cross-section (taken in parallel to the horizontal plane) to be described in more detail later.

(3) In the X-ray CT photos, an average cross-section area per single hydrophilic fiber was measured in the XY cross-section, the YZ cross-section and the XZ cross-section, respectively. The ratio of the cross-sectional area per single hydrophilic fiber in the XZ cross-section to the average cross-sectional area of the average fiber cross-sectional area per single hydrophilic fiber in the XY cross-section and the average cross-sectional area in YZ cross-section was calculated as the planar orientation index for the lower layer portion 21 or the upper layer portion 22.

(4) The planar orientation index when the absorbent matrix 18 contains neither the first superabsorbent polymer particles 107 nor the second superabsorbent polymer particles 307 was obtained by procedures as follows.

(4-1) A test piece for observation having a planar shape and a dimension of 20 x 20mm was subjected to laminography to obtain a laminogram of this test piece for observation without mechanically breaking the test piece (with the use of an CT scanner to be described in more detail).

Measurement and photographic device 3D measuring X-ray CT scanner: TDM-1000-IS/SP (manufactured by Yamato Scientific Co., Ltd.)

Image processing software 3D volume rendering software: VG-Studio MAX (manufactured by Nihon Visual Science, Inc.)

Measurement and photographic conditions: X-ray tube voltage of 30kv, tube current of 30microA, pixel count of 1024 pixel x 1024 pixel, and a size visual field set to the maximum scale in which the total thickness of the test piece for observation falls.

(4-2) The XY cross-section, the YZ cross-section and the XZ cross-section were obtained by BMP processing and the respective cross-section images were transferred to USB Digital Scale, the image analysis software manufactured by Scaler Corporation.

(4-3) BMP images of measurement sites were clipped out. Specifically, in the XY cross-section and the YZ cross-section, about 1/3 of the middle section of the test piece for observation (the lower layer portion 21 or the upper layer portion 22) in the thickness direction Y was clipped out and, in the XZ cross-section, a section of about 500 pixels x 500 pixels was clipped out.

(4-4) The images obtained in this manner were binarized so that the cross-sectional regions of the hydrophilic fibers on the respective cross-sections could be extracted.

(4-5) The lower limit for element extraction was set to 200micrometer'- under conversion to the cross-sectional area of the hydrophilic fiber and fibers having a cross-sectional area smaller than such cross-sectional area were precluded from the object the element extraction.

(4-6) The area occupancy factors of the hydrophilic fibers in the respective cross-sections were calculated and it was confirmed that the respective area occupancy factors in the XY cross-section, the YZ cross-section and the XZ cross-section are substantially equivalent.

(4-7) The average number of pixels was calculated from the cross-sections of the all hydrophilic fibers element-extracted on the respective cross-sections.

(4-8) The planar orientation index was calculated according to a formula as follows:

Planar orientation index = (average number of pixels in XZ cross-section)/{(average number of pixels in XY cross-section + average number of pixels in YZ cross-section)/2}

(5) As for the absorbent matrix 18 containing the first superabsorbent polymer particles 107 and/or the second superabsorbent polymer particles 307, the planar orientation indices were obtained according to procedures as follow. In this regard, in order to extract the fiber element range from the test piece for observation containing a mixture of the hydrophilic fibers and the superabsorbent polymer particles, the upper limit of the fiber element range was set. In the test piece for observation containing the mixture of the hydrophilic fibers and the superabsorbent polymer particles, after the element value of the superabsorbent polymer particles of which the cross-section was larger than the preset upper limit of the element range had been precluded, the average numbers of pixels and the planar orientation indices in the respective cross-sections were calculated.

(5-1) The same X-ray CT scanner and operating conditions thereof as those described in (4-1) were adopted.
(5-2) The same operating conditions as described in (4-2) were adopted.
(5-3) The same operationing conditions as described in (4-3) were adopted.
(5-4) The image obtained was binarized so that the cross-sectional regions of the hydrophilic fibers and the superabsorbent polymer particles could be extracted.
(5-5) The upper limit of the element extraction range was set to the number of pixels corresponding to 7000micrometer'- on the basis of the obtained image.
(5-6) The element value of the superabsorbent polymer particles was precluded from the upper limit of the element extraction range of the hydrophilic fibers.
(5-7) The lower limit for element extraction was set to 200micrometer$^2$ under conversion to the cross-sectional area of the hydrophilic fiber and fibers having a cross-sectional area smaller than such cross-sectional area were precluded from the object for the element extraction.
(5-8) In the cross-sections including both the hydrophilic fibers and the superabsorbent polymer particles, the area occupancy factors of the hydrophilic fibers were calculated and it was confirmed that the respective area occupancy factors in the XY cross-section, the YZ cross-section and the XZ cross-section are substantially equivalent.
(5-9) The average number of pixels was calculated from the cross-sections of the all hydrophilic fibers element-extracted on the respective cross-sections.
(5-10) The planar orientation index was calculated according to a formula as follows:

$$\text{Planar orientation index} = (\text{average number of pixels in XZ cross-section})/\{(\text{average number of pixels in XY cross-section} + \text{average number of pixels in YZ cross-section})/2\}$$

Evaluation Item 4: Movement of bodily fluids

(1) It is preferable for the sanitary napkin 11 that bodily fluids such as menstrual blood discharged thereon move to the lower layer portion 21 without staying in the upper layer portion 22 after having moved from the topsheet 12 to the upper layer portion 22 of the absorbent matrix 18. Specifically, if the amount of bodily fluids contained in the lower layer portion 21 is larger than the amount of bodily fluids contained in the upper layer portion 22 as a result of observation of the absorbent matrix 18 after bodily fluids have been discharged thereon, it can be considered that this absorbent matrix 18 is effective in alleviating an uncomfortable wet feeling which might be experienced by the wearer.
(2) On one hand, to verify such performance and effect of the absorbent matrix 18, a 70 x 160mm fragment of the absorbent matrix 18 was prepared and this fragment was separated into the upper layer portion 22 and the lower layer portion 21 and the masses (A1, B1 (g)) of the respective portions 22, 21 were obtained.
(3) On the other hand, a 70 x 160mm fragment of the absorbent matrix 18 was prepared and 0.3cc of artificial menstrual blood was dropped to the upper layer portion 22 of this fragment. In addition, 0.3cc of artificial menstrual blood was dropped on regions spaced apart 30mm or more from the first region. The operation of dropping 0.3cc of artificial menstrual blood was repeated every 10 seconds on a total of five regions. As artificial menstrual blood, an aqueous solution having a composition as follows was used:

Ion-exchanged water 1000g
Glycerin 40g
Carboxymethylcellulose-sodium(NaCMC) 8g
Sodium chloride (NaCl) 10g
Sodium acid carbonate(NaHCO$_3$) 4g

Pigment (Red No. 102) 8g
Pigment (Red No. 2) 2g
Pigment (Yellow No. 5) 2g

(4) Three minutes after the first drop of the artificial menstrual blood described in the above-mentioned section (3), the upper layer portion 22 was peeled off from the lower layer portion 21.
(5) The masses of the upper layer portion 22 and the lower layer portion 21 were measured with use of an electronic balance (A2, B2 (g)).
(6) The upper layer portion 22 and the lower layer portion 21 were dried for 2 hours in an oven at a temperature of 110°C and the masses thereof were measured after drying (A3, B3 (g)).
(7) Artificial menstrual blood retention (W3%) of the lower layer portion 21 was calculated according to the formulas as follows:

$$\text{Mass of actually dropped menstrual blood (W2)} = \{\text{mass of the upper layer portion before drying (A2)} + \text{mass of the lower layer portion before drying (B2)}\} - \text{mass of the absorbent matrix before dropping (A1 + B1)}$$

$$\text{The absorbent matrix's initial moisture content (W1)} = \{(\text{mass of the upper layer portion before drying (A2)} + \text{mass of the lower layer portion before drying (B2)}\} - \{\text{mass of the upper layer portion (A3) after drying} + \text{mass of the lower layer portion after drying (B3)}\} - \text{mass of actually dropped menstrual blood (W2)} \times \text{moisture percentage of artificial menstrual blood (\%)}$$

$$\text{The upper layer portion's initial moisture content (W1}_A) = \text{the absorbent matrix's initial moisture content (W1)} \times \{\text{mass of the upper layer portion's basis mass (BSA (g/m}^2\text{))}/\text{the absorbent matrix's basis mass (BSB (g/m}^2\text{))}.$$

$$\text{Mass of menstrual blood retained by the upper layer portion (W2}_A) = \text{mass of the upper layer portion before dried (A2)} - \text{mass of the upper layer portion after dried (A3)} - \text{the upper layer portion's initial moisture content (W1}_A).$$

$$\text{Mass of menstrual blood retained by the lower layer portion (W2}_B) = \text{mass of actually dropped menstrual blood (W2)} - \text{mass of menstrual blood retained by the upper layer portion (W2}_A).$$

$$\text{Percentage by mass of menstrual blood retained by the lower layer portion (W3\%)} = \{\text{mass of menstrual blood retained by the lower layer portion (W2}_B)/\text{mass of actually dropped menstrual blood (W2)}\} \times 100.$$

Evaluation Item 5: Sinking of the surface of the upper layer portion and spreading of menstrual blood in the upper layer portion and the lower layer portion

(1) If the upper layer portion of the absorbent matrix is of a relatively low density, menstrual blood, having moved through the topsheet to the absorbent matrix, will create a situation under cohesive force, i.e., intrinsic cohesive force of menstrual blood and its own mass, such that the portion of the absorbent matrix's surface wetted with menstrual blood sags more significantly than the remaining portion not wetted therewith and an amount of menstrual blood stays at this sagging portion. In consequence, this staying menstrual blood might make the wearer uncomfortable and/or contaminate the wearer's skin. To observe the degree of such sag occurring on the surface of the absorbent matrix, a 2D-Laser Displacement Gauge manufactured by Keyence Corporation, adapted to measure a change in the height of the absorbent matrix's surface, was used. This gauge is adapted to measure and to store 2D data concerning the surface profile of a nonwoven fabric or the like on the basis of laser reflected light.

(2) In the absorbent matrix 18, if menstrual blood quickly moves from the upper layer portion 22 to the lower layer portion 21 to draw apart from the wearer's skin, contamination of the wearer's skin with menstrual blood and a wet feeling experienced by the wearer will be alleviated. Also in the sanitary napkin 1 after menstrual blood has been completely absorbed therein, the presence of menstrual blood will be unnoticeable. In order that the absorbent matrix 18 is able to function in this manner, it is preferable that a menstrual blood spreading area is relatively small in the upper layer portion 22 and relatively large in the lower layer portion 21. Such spreading area was also observed as described below.

(3) Main devices of the measuring system:

> a. Sensor head LJ-G030 (manufactured by Keyence Corporation): used for laser transmission and as a measuring head.
> b. Controller LJ-G5000 (manufactured by Keyence Corporation): used for data accumulation and storage.
> c. Liquid crystal display monitor CA-MP81 (Keyence Corporation): used as a monitor.
> d. Power supply device and the additional LCD monitors.

(4) The sensor head described above in (3)-a is adapted to measure the height data over a rectilinear length of about 22mm at an error range of +/- 10mm. Setting the measuring pitch in the coordinates in the height direction to 0.033mm and without displacing the sensor head, coordinates before and after dropping of menstrual blood were measured. The measured coordinate data in the height direction was multiplied by the dimension of the measuring pitch and integrating the product to obtain cross-sectional change data in the menstrual blood absorbed portion of the absorbent matrix.

(5) For extraction of the coordinate data in the height direction, an application software LJ Navigator (version 1.5.1.0) adjunct to the aforementioned controller was used and for aggregate calculation and calculation of the cross-sectional change, the data were transferred to the spreadsheet software Excel 2003 manufactured by Microsoft Corporation.

(6) Details of the measuring procedure were as follow:

> a. The absorbent matrix 18 having the upper wrapping sheet 17 and the lower wrapping sheet 16 previously peeled off was cut to obtain a test piece of 100 x 100mm.
> b. The absorbent matrix 18 was set to a 2D-Laser Displacement Gauge manufactured by Keyence Corporation and irregularities on the surface were measured.
> c. 0.3cc of artificial menstrual blood was dropped for 1 second to the region of the absorbent matrix 18 corresponding to a central region of the laser measuring range.
> d. Twenty seconds after dropping of the artificial menstrual blood, the surface irregularities were measured at the same region as the region in which the measurement had been conducted in the above-mentioned step b.
> e. Immediately after the measurement in the above-mentioned step d, dimensions of the zone of the absorbent matrix's surface wetted with the artificial menstrual blood having passed therethrough were measured in two directions being orthogonal to each other.
> f. The dimensions in two directions having been measured in the above-mentioned step e were ellipse-converted to obtain a surface spreading area on the absorbent matrix 18. Result of the calculation is indicated in TABLE 1 as "spreading area on upper surface".
> g. The coordinates having been obtained in the above-mentioned step b were subtracted from the coordinates having been obtained in the above-mentioned step f and the difference was multiplied by the measuring pitch in the measuring width direction. The product was integrated to record the cross-sectional change before and after absorption of the menstrual blood. The result is indicated in TABLE 1 as "sagging area".
> h. Three minutes after the above-mentioned step c, the dimensions of the range in which the presence of the artificial menstrual blood can be visually recognized as viewed from the lower side of the absorbent matrix 18 as measured in two directions being orthogonal to each other. The measured dimensions were ellipse-converted to obtain the spreading area of the menstrual blood as viewed from the lower side. The result is indicated in TABLE 1 as "spreading area on lower surface".

Evaluation Item 6: Q-max value after absorption of menstrual blood

(1) To evaluate warm-cold sensations experienced by the wearer of the sanitary napkin 11 due to movement of the menstrual blood in the absorbent matrix 18, the Q-max value after the artificial menstrual blood has been dropped to the absorbent matrix 18 was measured.

(2) For this measurement, a KES-F7 High Accuracy & High-Speed Thermal Property Measuring Device THERMOLABO II Model (manufactured by KATO TECH CO., LTED.) was used. Ten seconds after an amount of artificial menstrual blood depending on the thickness of the absorbent matrix 18 had been dropped to the absorbent matrix 18, a pure copper plate as a sensor of THERMOLABO II was put in contact with the surface of the upper layer portion 22 in the absorbent matrix 18 and movement of heat quantity between the upper layer portion 22 and the copper plate was measured to obtain an index representing the warm-cold sensation experienced by the wearer when the wearer's skin comes into contact with the upper layer portion 22.

(3) The term "Q-max value" refers to the peak value of a heat quantity transferred from the heat storing pure copper plate having an area of $9cm^2$ and a mass of 9.79g (heat capacity of 0.41855J/°C) to the upper layer portion 22, measured immediately after the pure copper plate was put in contact with the upper layer portion 22 (the heat quantity reaches the peak value about 0.2 seconds after contact under the effect of a filter). According to the printed explanation offered by the manufacturer of THERMOLABO II, the heat transfer considered to pertain to the warm-cold sensation experienced by the wearer at a moment of contact between the wearer's skin and the upper layer portion 22 can be simulated by this filter. According also to the printed explanation, at higher Q-max values, the cold sensation experienced by the wearer becomes more significant and, at lower Q-max values, the warm feeling experienced by the wearer becomes more significant. The Q-max value is considered to be proportional to a difference delta T between an initial temperature $T_0$ of the copper plate and a temperature $T_S$ of the upper layer portion 22 and, in addition, to depend on a contact pressure.

(4) Measurement of Q-max value was conducted in a test laboratory conditioned at a temperature of 20°C and a relative humidity of 60%. The matrixes 18, the measuring devices, the artificial menstrual blood, etc. were left at rest in this test laboratory for 12 hours or longer to condition them. Under such conditioning, the temperature $T_S$ of the upper layer portion 22 in each of the absorbent matrixes 18 was set to 20°C.

(5) A quantity of the artificial menstrual blood to be dropped on to the absorbent matrix 18 was regulated to be 0.5cc per 3mm thickness of the absorbent matrix 18. Specifically, 0.6cc of the artificial menstrual blood was dropped to an absorbent matrix 18 having a thickness of 3.8mm and 0.78cc of the artificial menstrual blood was dropped to an absorbent matrix 18 having thickness of 4.7mm.

(6) The sensor's contact pressure to the absorbent matrix 18 was set to $30g/cm^2$ on the basis of a pressure exerted on the sanitary napkin when the napkin wearer is in seated posture.

(7) The measurement was conducted by following the procedures described below.

a. The copper plate in THERMOLABO II was heated so that the copper plate may have an initial temperature $T_0$ of 30°C.

b. Each of the absorbent structures 14 was cut to a size of 100 x 100mm and the lower wrapping sheet 16 and the upper wrapping sheet 17 were peeled off to take out the absorbent matrix 18. The absorbent matrixes 18 obtained in this manner were used as test pieces for measurement after conditioning.

c. Quantities of the artificial menstrual blood, predetermined depending on the thickness of the respective test pieces, were dropped to central regions thereof.

d. Ten seconds after the start of dropping, the central region of the sensor in THERMOLABO II was aligned and put in contact with the central regions of the respective test pieces on which the artificial menstrual blood was dropped and thereby the Q-max values of the respective test pieces under the predetermined contact pressure were measured.

e. For evaluation of the absorbent matrix, the measurement was conducted on three test piece and the respective measurement results were averaged to obtain the Q-max value.

(8) The measurement result is indicated in TABLE 1 as "Q-max".

(Example 3)

An absorbent matrix according to Example 3 was made by following the process illustrated in Fig. 3 under the same conditions as for Example 1, except that NB-416 Fluff wood pulp of Weyerhauser was replaced by Supersoft of International Paper Corporation. The evaluation result obtained for Example 3 is indicated in TABLE 1. In this regard, the average fiber length of Supersoft was 2.37mm.

(Examples 4, 5)

According to Example 4, the absorbent matrix was made by following the process illustrated in Fig. 3 under the same conditions as for Example 1, except that superabsorbent polymer particles UG860D, manufactured by Sumitomo Seika Chemicals Co., Ltd., were mixed at rates of 133.3g/m$^2$ and 66.7g/m$^2$ into the lower layer portion and the upper layer portion, respectively.

According to Example 5, the absorbent matrix was made by following the process as for Example 4, except that the superabsorbent polymer particles UG860D used in Example 4 were replaced by the superabsorbent polymer particles manufactured by San-Dia Polymers, Ltd. in Japan.

The evaluation results for the matrixes according to Examples 4, 5 are indicated in TABLE 1.

(Example 6)

An absorbent matrix according to Example 6 was made by following the same process as for Example 1, except that the superabsorbent polymer particles UG860D were mixed in the lower layer portion at a rate of 200g/m$^2$. The evaluation result for the absorbent matrix according to Example 6 is indicated in TABLE 1.

(Example 7)

An absorbent matrix according to Example 7 was made under the same conditions as for Example 1 except that, in the fourth step IV of the process illustrated in Fig. 3, a spacing distance CL2 larger than the spacing distance CL2 adopted by Example 1 was adopted. The evaluation result for the absorbent matrix according to Example 7 is indicated in TABLE 1. The spacing distance CL2 is indicated in TABLE 1 as "dimension (mm) of compression-molded upper and lower layer portions". With the dimension of the spacing distance CL2 enlarged in this manner, the specific volume of the upper layer portion is correspondingly enlarged and the planar orientation index thereof is made smaller.

(Example 8)

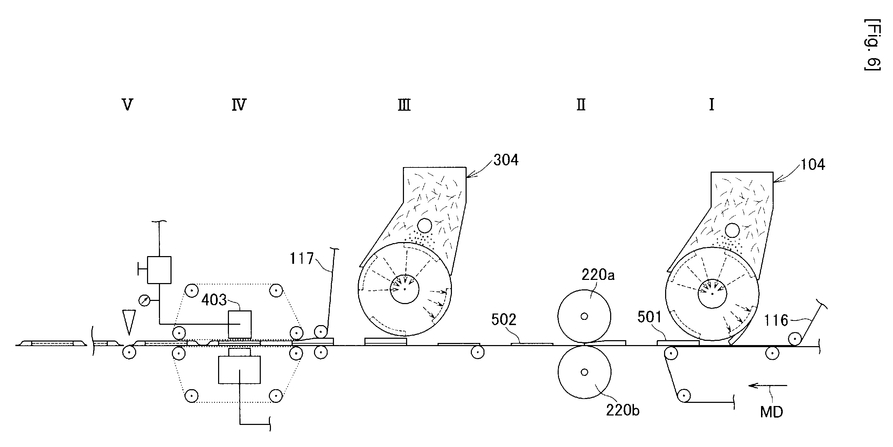

The absorbent matrix according to Example 8 was made by following the process illustrated in Fig. 6. The process illustrated in Fig. 6 is the same as the process illustrated in Fig. 3 except that paired press rolls 220a, 220b are used in the second step II. The first aggregate 501 is the same as that obtained in the process illustrated in Fig. 3. This first aggregate 501 is compressed by the press rolls 220a, 220b to obtain the second aggregate 502 having a thickness substantially the same as the thickness of the second aggregate in Example 1.

The evaluation result for the absorbent matrix according to Example 8 is indicated in TABLE 1. Compared to the absorbent matrix according to Example 1, the planar orientation index in the lower layer portion in this absorbent matrix is larger than the planar orientation index in Example 1 and it is considered that this relatively large planar orientation index in the lower layer portion enlarged a difference of the planar orientation indices of the upper layer portion and the lower layer portion.

(Comparative Examples)

(Comparative Examples 1, 2)

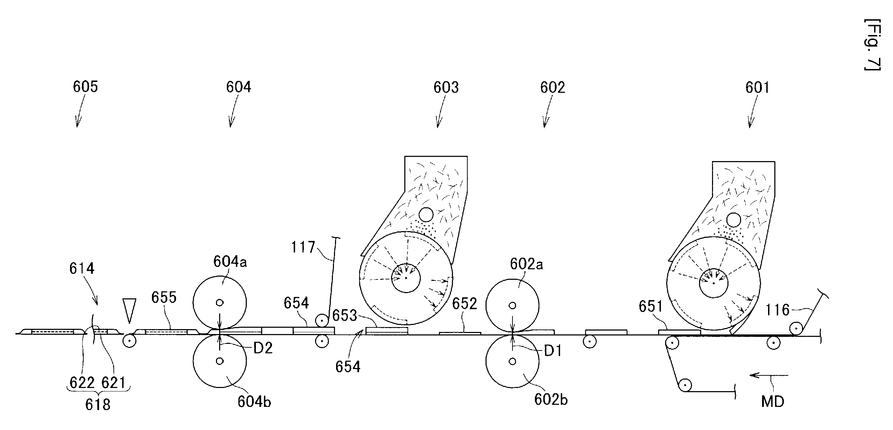

Fig. 7 illustrates a process for manufacturing the matrixes according to Comparative Example 1 for comparison with the matrixes according to Examples 1, 2. In this regard, these processes are distinguished from the process illustrated in Fig. 3 only in second and fourth steps 602, 604 and the remaining first, third and fifth steps 601, 603, 605 are the same as the first, third and fifth steps I, III, V in Fig. 3. In the second step 602 illustrated in Fig. 7, the first block 203 and the first box 204 in the second step in Fig. 3 were replaced by paired press rolls 602a, 602b adapted to be used under no temperature control to compress a first aggregate 651 and thereby to form a second aggregate 652. The first aggregate 651 formed in a first step 601 is similar to the first aggregate 501 in Fig. 3 and this first aggregate 651 is compressed by press rolls 602a, 602b so as to obtain the second aggregate 652 having substantially the same thickness as the first aggregate 501 in Fig. 3.

In the fourth step 604 illustrated in Fig. 7, a fourth aggregate 655 having been formed downstream in the third step 603 is compressed by paired press rolls 604a, 604b to form a fifth aggregate 655. The fourth aggregate 654 is compressed by the paired press rolls 604a, 604b so as to obtain the fifth aggregate 655 having substantially the same thickness as that of the fifth aggregate 505 in Fig. 3. The fifth aggregate 655 corresponds to the matrixes 618 according to Comparative Examples 1 and 2.

In the fifth step 605 illustrated in Fig. 7, the carrier web 116 and the cover web 117 were cut along the cutting line between each pair of the fifth aggregates 655 being adjacent in the machine direction MD, to obtain the individual absorbent structure 614 containing therein the absorbent matrix 618. The absorbent matrix 618 includes the lower layer portion 621 defined by the first aggregate 651 and the upper layer portion 622 defined by the third aggregate 653.

In the process illustrated in Fig. 7, the matrixes according to Comparative Examples 1 and 2 were made under the conditions as follows:

(1) Hydrophilic fiber used in the first and third steps 601, 603: NB-416 manufactured by Weyerhauser Co., Ltd.
(2) The carrier web 116 and the cover web 117: The same carrier web 116 and cover web 117 as those used in the process illustrated in Fig. 3.
(3) Used amount of hydrophilic fibers:

(Comparative Example 1)

The first aggregate 651 (the lower layer portion 621 of the absorbent matrix 618): 200g/m$^2$
The third aggregate 653 (the upper layer portion 622 of the absorbent matrix 618: 100g/m$^2$

(Comparative Example 2)

The first aggregate 651: 200g/m$^2$
The second aggregate 652: 200g/m$^2$

The evaluation results of the matrixes according to Comparative Examples 1, 2 on the same evaluation item for the matrixes according to Examples 1, 2 are indicated in TABLE 1.

(Comparative Example 3)

An absorbent matrix according to Comparative Example 3 was made under the same conditions as for Comparative Example 1 except that NB-416 Fluff wood pulp was replaced by Supersoft manufactured by International Paper Corporation. The absorbent matrix according to Comparative Example 3 was compared to the absorbent matrix according to Example 3. The evaluation result is indicated in TABLE 1.

(Comparative Examples 4, 5)

The matrixes to be compared with the matrixes according to Examples 4 and 5 were made as described below.
According to Comparative Example 4, an absorbent matrix was made under the same conditions as for Comparative Example 1, except that superabsorbent polymer particles UG860D manufactured by Sumitomo Seika Chemicals Co., Ltd. were mixed into the lower layer portion and the upper layer portion of the absorbent matrix according to Comparative Example 1 at a rate of 133.3g/m$^2$ and 66.7g/m$^2$, respectively.
According to Comparative Example 5, an absorbent matrix was made under the same conditions as that for Comparative Example 4 except that the superabsorbent polymer particles UG860D used in Comparative Example 4 were replaced by superabsorbent polymer particles UP270. The evaluation results for the matrixes according to Comparative Examples 4 and 5 are indicated in TABLE 1.

(Comparative Example 6)

An absorbent matrix according to Comparative Example 6, to be compared with the absorbent matrix according to Example 6, was made in the manner as follows. Except that superabsorbent polymer particles UG860D were mixed into the lower layer portion of the absorbent matrix according to Comparative Example 1 at a rate of 200g/m$^2$, the absorbent matrix according to Comparative Example 6 was made under the same conditions as for Comparative Example 1. The evaluation result is indicated in TABLE 1.

(Comparative Example 7)

An absorbent matrix according to Comparative Example 7, to be compared to the absorbent matrix according to Example 7, was made in the manner as follows. Specifically, except that the spacing distance D2 having a dimension larger than the dimension of the spacing distance D2 of the paired press rolls 604a, 604b in the fourth step for manufacturing the absorbent matrix according to Comparative Example was adopted, the absorbent matrix according to Comparative Example 7 was made under the same conditions as for Comparative Example 1. The evaluation result for the absorbent matrix according to Comparative Example 7 is indicated in TABLE 1. In this regard, the spacing distance D2 is indicated in TABLE 1 as "dimension (mm) of compression-molded upper and lower layer portions".

(Comparative Example 8)

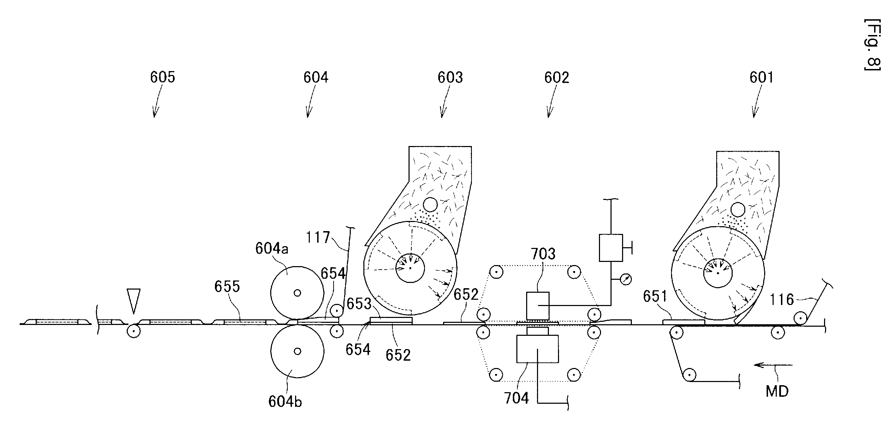

The absorbent matrix according to Comparative Example 8, to be compared with the absorbent matrix according to Example 8, was made in the manner as follows. Specifically, a process as illustrated in Fig. 8 was used to make the absorbent matrix according to Comparative Example 8. The process illustrated in Fig. 8 includes first through fifth steps 601 through 605 and similar to the process illustrated in Fig. 7 except that the same step as the second step II in Fig. 3 is adopted as the second step 602. In the second step 602 illustrated in Fig. 8, in the course of passing between a high pressure steam jet block 703 and a steam suction box 704, the first aggregate 651 is subjected to a high pressure steam jet under the same conditions those under which the second aggregate 501 according to Example 1 is subjected to the high pressure steam jet and thereby the first aggregate 651 is obtained. In the process illustrated in Fig. 8, the fourth aggregate 654 is compressed by the paired press rolls 604a, 604b and thereby the fifth aggregate 655, i.e., the absorbent matrix according to Comparative Example 8, is obtained. The absorbent matrix according to Comparative Example 8, is compressed to substantially the same thickness as that of the absorbent matrix according to Example 8. The evaluation result for the absorbent matrix according to Comparative Example 8 is indicated in TABLE 1.

[Table 1]

| Sample | Portions of absorbent matrix | 1st,2nd hydrophilic fibers | Basis mass of 1st,2nd hydrophilic fibers (g/m²) | Trade name of used SAP | Basis mass of SAP particles (g/m²) | Compressive means | Compression-molded upper & lower layer portions dimension (mm) | Compression-molded lower layer portions total dimension (mm) | Eval.1 Thickness of respective layers (mm) | Eval.2 Basis mass of respective layers (g/m²) | Eval.2 Specific volume | Eval.2 Difference of specific volume between upper & lower layers | Eval.3 Planar orientation index | Eval.3 Difference of planar orientation index between upper & lower layers | Eval.4 Rate of movement to lower layer (%) | Eval.4 Spreading area in upper layer (mm²) | Eval.4 Spreading area in lower layer (mm²) | Eval.5 Sagging area (mm²) | Eval.6 Q max |
|---|---|---|---|---|---|---|---|---|---|---|---|---|---|---|---|---|---|---|---|
| Ex.1 | Upper layer | NB416 | 100 | — | — | Steam jets | 1.10 | 1.85 | 1.8 | 96.79 | 18.6 | 7.77 | 1.15 | 0.40 | 77.98 | 77.63 | 310.21 | 35.90 | 0.201 |
| | Lower layer | NB416 | 200 | — | — | | | | 2.0 | 184.64 | 10.8 | | 1.55 | | | | | | |
| Ex.2 | Upper layer | NB416 | 200 | — | — | Steam jets | 1.10 | 2.60 | 2.9 | 174.46 | 16.6 | 6.7 | — | | 29.79 | 88.09 | 24.96 | 30.61 | 0.085 |
| | Lower layer | NB416 | 200 | — | — | | | | 1.8 | 181.16 | 9.9 | | — | | | | | | |
| Com-Ex.1 | Upper layer | NB416 | 100 | — | — | Pressurize | 0.35 | 0.70 | 1.6 | 92.50 | 17.3 | 6.14 | 1.40 | 0.12 | 45.98 | 100.39 | 20.26 | 49.41 | 0.836 |
| | Lower layer | NB416 | 200 | — | — | | | | 2.0 | 179.20 | 11.2 | | 1.52 | | | | | | |
| Com-Ex.2 | Upper layer | NB416 | 200 | — | — | Pressurize | 0.35 | 2.20 | 3.0 | 174.64 | 17.2 | 5.9 | — | | 9.87 | 111.10 | 0.00 | 34.64 | 0.392 |
| | Lower layer | NB416 | 200 | — | — | | | | 2.1 | 185.27 | 11.3 | | — | | | | | | |
| Ex.3 | Upper layer | Supersoft | 100 | — | — | Steam jets | 1.10 | 1.85 | 1.8 | 91.88 | 19.6 | 11.3 | — | | 72.53 | 92.53 | 211.02 | 15.06 | 0.184 |
| | Lower layer | Supersoft | 200 | — | — | | | | 1.7 | 205.71 | 8.3 | | — | | | | | | |
| Ex.4 | Upper layer | NB416 | 100 | UG860 | 66.7 | Steam jets | 1.00 | 2.00 | 1.6 | 155.80 | 10.3 | 5.13 | 1.25 | 0.34 | 71.96 | 221.50 | 271.61 | 10.40 | 0.181 |
| | Lower layer | NB416 | 200 | UG860 | 133.3 | | | | 2.1 | 408.93 | 5.1 | | 1.59 | | | | | | |
| Ex.5 | Upper layer | NB416 | 100 | UP270 | 66.7 | Steam jets | 1.00 | 2.00 | 2.1 | 120.68 | 17.4 | 11.99 | 1.21 | 0.44 | 76.80 | 218.92 | 274.68 | 5.42 | 0.154 |
| | Lower layer | NB416 | 200 | UP270 | 133.3 | | | | 2.1 | 387.77 | 5.4 | | 1.65 | | | | | | |
| Ex.6 | Upper layer | NB416 | 100 | UG860 | 0 | Steam jets | 0.80 | 1.60 | 1.5 | 108.64 | 13.8 | 9.71 | 1.19 | 0.55 | 83.04 | 217.34 | 296.88 | -4.39 | 0.125 |
| | Lower layer | NB416 | 200 | UG860 | 200 | | | | 1.9 | 464.02 | 4.1 | | 1.74 | | | | | | |
| Com-Ex.3 | Upper layer | Supersoft | 100 | — | — | Press rolls | 0.34 | 0.65 | 1.5 | 92.68 | 16.2 | 5.3 | — | | 64.27 | 117.26 | 4.24 | 39.35 | 0.400 |
| | Lower layer | Supersoft | 200 | — | — | | | | 2.0 | 184.20 | 10.9 | | — | | | | | | |
| Com-Ex.4 | Upper layer | NB416 | 100 | UG860 | 66.7 | Pressurize | 0.45 | 1.25 | 2.4 | 132.32 | 18.1 | 11.70 | 1.55 | 0.07 | 49.78 | 245.94 | 145.89 | 25.55 | 0.387 |
| | Lower layer | NB416 | 200 | UG860 | 133.3 | | | | 2.0 | 310.45 | 6.4 | | 1.62 | | | | | | |
| Com-Ex.5 | Upper layer | NB416 | 100 | UP270 | 66.7 | Pressurize | 0.53 | 1.21 | 1.9 | 145.45 | 13.1 | 7.88 | 1.46 | 0.13 | 60.87 | 242.72 | 159.72 | 21.67 | 0.368 |
| | Lower layer | NB416 | 200 | UP270 | 133.3 | | | | 1.8 | 347.41 | 5.2 | | 1.59 | | | | | | |
| Com-Ex.6 | Upper layer | NB416 | 100 | UG860 | 0 | Pressurize | 0.53 | 0.97 | 1.2 | 82.14 | 14.6 | 10.44 | 1.42 | 0.18 | 62.53 | 229.38 | 180.75 | 15.89 | 0.314 |
| | Lower layer | NB416 | 200 | UG860 | 200 | | | | 1.6 | 384.11 | 4.2 | | 1.6 | | | | | | |
| Ex.7 | Upper layer | NB416 | 100 | — | — | Steam jets | 1.10 | 2.10 | 2.0 | 85.00 | 23.5 | 14.08 | 1.09 | 0.63 | 85.43 | 70.34 | 365.17 | 24.59 | 0.091 |
| | Lower layer | NB416 | 200 | — | — | | | | 1.9 | 201.00 | 9.5 | | 1.72 | | | | | | |
| Com-Ex.7 | Upper layer | NB416 | 100 | — | — | Pressurize | 0.35 | 1.80 | 2.1 | 96.00 | 21.9 | 13.02 | 1.31 | 0.14 | 51.35 | 94.18 | 100.36 | 46.78 | 0.749 |
| | Lower layer | NB416 | 200 | — | — | | | | 1.7 | 192.00 | 8.9 | | 1.45 | | | | | | |
| Ex.8 | Upper layer | NB416 | 100 | — | — | Steam jets | 0.35 | 2.05 | 1.8 | 97.00 | 18.6 | 10.11 | 1.13 | 0.57 | 81.39 | 75.34 | 344.23 | 33.04 | 0.117 |
| | Lower layer | NB416 | 200 | — | — | Pressurize | | | 1.8 | 213.00 | 8.5 | | 1.70 | | | | | | |
| Com-Ex.8 | Upper layer | NB416 | 100 | — | — | Pressurize | 1.10 | 0.70 | 2.0 | 110.00 | 18.2 | 8.40 | 1.56 | -0.05 | 36.62 | 112.94 | 0.50 | 55.32 | 1.330 |
| | Lower layer | NB416 | 200 | — | — | Steam jets | | | 1.8 | 184.00 | 9.8 | | 1.51 | | | | | | |

In TABLE 1, the evaluation of the planar orientation index indicates that, while no remarkable difference is observed in the planar orientation indices between the matrixes according to the Examples and the matrixes according to the Comparative Examples so far as the lower layer portions are concerned, the planar orientation indices are smaller in the matrixes according to the Examples than in the matrixes according to the Comparative Examples so far as the upper layer portions are concerned. In other words, the difference between the planar orientation indices of the upper and lower layer portions is more significant in the matrixes according to Examples than in the matrixes according to the Comparative Examples. Based on such evaluation results, it can be concluded that it is effective to subject the hydrophilic fiber aggregate of the upper layer portion to a high pressure steam jet in order to orient the hydrophilic fibers in the thickness direction of the absorbent matrix.

With an absorbent matrix in which the difference in the planar orientation index is relatively large between the upper layer portion and the lower layer portion, as is the case in the matrixes according to the Examples , a possibility that bodily fluids such as menstrual blood might spread in the upper layer portion or stay therein can be restricted, on one hand, and such bodily fluids can smoothly spread over a wide range in the lower layer portion, on the other hand. Consequentially, for example, in the absorbent matrix (See, for example, Example 4) in which the planar orientation index in the upper layer portion is less than 1.30 and the planar orientation index in the lower layer portion is at least 0.3 larger than the planar orientation index in the upper layer portion, the Q-max value of this absorbent matrix is apt to be smaller than the Q-max value of the absorbent matrix according to the Comparative Examples. A bodily fluid absorbent wearing article such as a sanitary napkin in which the absorbent matrix has a relatively small Q-max value is preferable as the wearing article since the wearer can be substantially free from an uncomfortable cold sensation due to bodily fluids absorbed by the article.

In the process of Fig.3 for manufacturing the absorbent matrix 18, the first aggregate 501 was placed on the carrier web 116 and, in this state, the first aggregate 501 was subjected to a high pressure steam jet. Use of the carrier web 116 in this manner is preferable in view of preventing the first hydrophilic fibers 105 and/or the first superabsorbent polymer particles 107 from falling off from the first aggregate 501. In this regard, however, if there is no possibility that the first hydrophilic fibers 105 and/or the first superabsorbent polymer particles 107 may fall off from the first aggregate 501, it may be possible to load the first aggregate 501 directly onto an air-permeable conveyor belt instead of placing the first aggregate 501 on the carrier web 116 and to transport it through the second step II. In this case, the carrier web 116 may be supplied between the second step II and the third step III or between the third step III and the fourth step IV or between the fourth step IV and the fifth step V. It is also possible to supply the cover web 117 between the fourth step IV and the fifth step V.

In the process for manufacturing the absorbent matrix 18 illustrated by way of example in Fig. 6, the first aggregate 501 is placed on the carrier web 116 and compressed in the second step II by the paired rolls 220a, 220b. In this case also, the carrier web 116 serves to prevent the first hydrophilic fibers 105 and/or the first superabsorbent polymer particles 107 from falling off from the first aggregate 501. It should be noted here that it is also possible to load the first aggregate 501 directly onto the conveyor belt instead of placing on the carrier web 116 and to transport the first aggregate together with the conveyor belt through the second step II and the fourth step IV. In this case, the carrier web 116 may be supplied between the second step II and the third step III or between the third step III and the fourth step IV or between the fourth step IV and the fifth step V.

The first aspect of the present invention described hereinabove may be arranged in at least the following features:

(I) A bodily fluid absorbent matrix including an aggregate of bodily fluid absorbent material interposed between a liquid-permeable first sheet lying on the skin-facing side in a bodily fluid absorbent wearing article and a liquid-permeable or liquid-impermeable second sheet lying on a garment-facing side opposite to the skin-facing side, wherein:

the absorbent matrix includes hydrophilic fibers serving as the bodily fluid absorbent material;
the absorbent matrix has a density gradient so as to increase from the first sheet toward the second sheet in a thickness direction of the absorbent matrix; and
a planar orientation index of the hydrophilic fibers increases in the thickness direction.

(i) The absorbent matrix has an upper layer portion and a lower layer portion overlapping each other in the thickness direction so that the upper layer portion faces the first sheet and the lower layer portion faces the second sheet;
the density of the upper layer portion is lower than that of the lower layer portion; and
the planar orientation index in the upper layer portion is less than 1.30 and the planar orientation index in the lower layer portion is at least 0.3 larger than the planar orientation index in the upper layer portion.
The aspect described in the above items (I) and (i) may include at least the following embodiments, which may be taken in isolation or in combination with one another:
(ii) The hydrophilic fibers have an average fiber length of 15mm or less.
(iii) The absorbent matrix contains at least fluff wood pulp as the hydrophilic fibers.
(iv) The absorbent matrix contains superabsorbent polymer particles and fluff wood pulp as the hydrophilic fibers.

The second aspect of the present invention described hereinabove may be arranged in at least the following features:

(II) A method for manufacturing an absorbent matrix including an aggregate of bodily fluid absorbent material interposed between a liquid-permeable first sheet lying on the skin-facing side in a bodily fluid absorbent wearing article and a liquid-permeable or liquid-impermeable second sheet lying on the side of garment-facing side opposite to the skin-facing side, wherein
the method includes at least the steps as follows:

(1) a step of providing the matrix with: an upper layer portion and a lower layer portion which are stacked on each other in the thickness direction and have the same thickness; and the aggregate including hydrophilic fibers as the bodily fluid absorbent material, and a plurality of first aggregates each formed of a portion of the bodily fluid absorbent material intended to form the lower layer portions are arranged intermittently in the machine direction and transported in the machine direction;

(2) a step of interposing the respective first aggregates between a pair of air-permeable first support means running in the machine direction between a first nozzle array for high pressure steam jets and a first suction port for suction of steam so that the respective first aggregates are subjected to high pressure steam jets from the first nozzle array under the suction effect of the first suction port to obtain second aggregates corresponding to the first aggregates compressed in the thickness direction thereof;

(3) a step of stacking a plurality of third aggregates of bodily fluid absorbent material intended to form the upper layer portions on the respective second aggregates to obtain fourth aggregates; and

(4) a step of interposing the fourth aggregates between a pair of air-permeable second support means running in the machine direction between a second nozzle array for high pressure stream jets and a second suction port for suction of steam facing each other so that the third aggregates in the fourth aggregates are subjected to high pressure steam jets from the second nozzle array under the suction effect from the second suction port to compress the fourth aggregates and thereby to obtain fifth aggregates from the fourth aggregates.

The aspects described in the above item (II) may include at least the following embodiments, which may be taken in isolation or in combination with one another:

(i) The fourth aggregate inclusive of the second aggregate placed on an air permeable first sheet and inclusive also of the third aggregate covered with an air-permeable second sheet is interposed between a pair of the second support means.

(ii) The first aggregate placed on an air-permeable first sheet is interposed together with the first sheet between a pair of the first support means.

(iii) The fourth aggregate inclusive of the third aggregate covered with an air-permeable second sheet is interposed together with the second sheet between a pair of the second support means.

(iv) The first support means and the second support means are mesh conveyor belts.

(v) The hydrophilic fibers have a fiber length of 15mm or less.

(vi) Instead of the first nozzle array and the first suction port, a pair of press rolls is used to compress the first aggregate.

(vii) The absorbent matrix contains at least fluff wood pulp as the hydrophilic fibers.

(viii) The absorbent matrix contains superabsorbent polymer particles and fluff wood pulp as the hydrophilic fibers.

The described aspects and/or embodiments provide one or more of the following effects:

(a) The absorbent matrix according to the first aspect of this invention has a density gradient increasing from the first sheet to the second sheet. With such a density gradient, the bodily fluids first absorbed by the first sheet move toward the second sheet and thereby move away from the skin of the wearer of the bodily fluid absorbent wearing article. In consequence, it is possible to restrict a wet feeling experienced by the wearer due to the bodily fluids. Furthermore, in the absorbent matrix of this invention, the planar orientation index of the hydrophilic fibers changes so as to increase in the direction from the first sheet toward the second sheet so that the hydrophilic fibers are apt to be oriented predominantly in the thickness direction in the upper layer portion in the vicinity of the first sheet and apt to be oriented predominantly in the planar direction being orthogonal to the thickness direction in the lower layer portion in the vicinity of the second sheet. In consequence, bodily fluids absorbed by the absorbent matrix from the side of the first sheet are apt to move predominantly in the thickness direction in the upper layer portion, but spreading thereof in the planar direction is restricted and, in the lower layer portion, bodily fluids are apt to spread predominantly in the planar direction, but movement thereof in the thickness direction is restricted. Spreading of bodily fluids in the planar direction is restricted in the vicinity of the first sheet and thereby the bodily fluid absorbent wearing article using such an absorbent matrix makes it possible to prevent a problem that bodily fluids discharged on the article might cause the wearer to experience a wet feeling over a wide range of the wearing article's surface.

(b) In the manufacturing process according to the second aspect of this invention, the first aggregate of the bodily fluid absorbent material adapted to define the lower layer portion of the absorbent matrix is subjected to high pressure steam jets, thereafter the third aggregate of the bodily fluid absorbent material is overlapped on the first aggregate and this third aggregate is subject to high pressure steam jets to obtain the lower layer portion having a large planar orientation index and the upper layer portion having a small planar orientation index.

## Claims

1. A bodily fluid absorbent matrix (18) including an aggregate of bodily fluid absorbent material interposed between a liquid-permeable first sheet (17) lying on the skin-facing side in a bodily fluid absorbent wearing article and a liquid-permeable or liquid-impermeable second sheet (16) lying on a garment-facing side opposite to the skin-facing side, wherein:

   the absorbent matrix includes hydrophilic fibers serving as the bodily fluid absorbent material;
   the absorbent matrix has a density gradient so as to increase from the first sheet toward the second sheet in a thickness direction of the absorbent matrix;
   a planar orientation index of the hydrophilic fibers increases in the thickness direction;
   the absorbent matrix (18) has an upper layer portion (22) and a lower layer portion (21) overlapping each other in the thickness direction so that the upper layer portion faces the first sheet and the lower layer portion faces the second sheet;
   the density of the upper layer portion is lower than that of the lower layer portion; and
   the planar orientation index in the upper layer portion is less than 1.30 and the planar orientation index in the lower layer portion is at least 0.3 larger than the planar orientation index in the upper layer portion.

2. The absorbent matrix defined by claim 1, wherein the hydrophilic fibers have an average fiber length of 15mm or less.

3. The absorbent matrix defined by claim 1 or 2, wherein the absorbent matrix contains at least fluff wood pulp as the hydrophilic fibers.

4. The absorbent matrix defined by any one of claims 1 to 3, wherein the absorbent matrix contains superabsorbent polymer particles and fluff wood pulp as the hydrophilic fibers.

5. A method for manufacturing a bodily fluid absorbent matrix (18) including an aggregate of bodily fluid absorbent material interposed between a liquid-permeable first sheet (17) lying on the skin-facing side in a bodily fluid absorbent wearing article and a liquid-permeable or liquid-impermeable second sheet (16) lying on the side of garment-facing side opposite to the skin-facing side, wherein
the method includes at least the steps as follows:

   (1) a step of providing the absorbent matrix with: an upper layerportion (22) and a lower layer (21) portion which are stacked on each other in the thickness direction and have the same thickness; and the aggregate including hydrophilic fibers (105, 305) as the bodily fluid absorbent material, and a plurality of first aggregates (501) each formed of a portion of the bodily fluid absorbent material intended to form the lower layer portions are arranged intermittently in the machine direction and transported in the machine direction;
   (2) a step of interposing the respective first aggregates (501) between a pair of air-permeable first support means (201, 202) running in the machine direction between a first nozzle array (203) for high pressure steam jets and a first suction port (204) for suction of steam so that the respective first aggregates are subjected to high pressure steam jets from the first nozzle array under the suction effect of the first suction port to obtain second aggregates (502) corresponding to the first aggregates compressed in the thickness direction thereof;
   (3) a step of stacking a plurality of third aggregates (503) of bodily fluid absorbent material intended to form the upper layer portions on the respective second aggregates to obtain fourth aggregates (504); and
   (4) a step of interposing the fourth aggregate between a pair of air-permeable second support means (401, 402) running in the machine direction between a second nozzle array (403) for high pressure stream jets and a second suction port (404) for suction of steam facing each other so that the third aggregates in the fourth aggregates are subjected to high pressure steam jets from the second nozzle array under the suction effect from the second suction port to compress the fourth aggregates and thereby to obtain fifth aggregates from the fourth aggregates (505).

6. The method defined by claim 5, wherein the fourth aggregate inclusive of the second aggregate placed on an air-permeable first sheet (116) and inclusive also of the third aggregate covered with an air-permeable second sheet (117) is interposed between a pair of the second support means.

7. The method defined by claim 5, wherein the first aggregate placed on an air-permeable first sheet (116) is interposed together with the first sheet between a pair of the first support means.

8. The method defined by claim 5, wherein the fourth aggregate inclusive of the third aggregate covered with an air-permeable second sheet (117) is interposed together with the second sheet between a pair of the second support means.

9. The method defined by any one of claims 5 to 8, wherein the first support means and the second support means are mesh conveyor belts.

10. The method defined by any one of claims 5 to 9, wherein the hydrophilic fibers have a fiber length of 15mm or less.

11. The method defined by any one of claims 5 to 10, wherein, instead of the first nozzle array and the first suction port, a pair of press rolls is used to compress the first aggregate.

12. The method defined by any one of claims 5 to 11, wherein the absorbent matrix contains at least fluff wood pulp as the hydrophilic fibers.

13. The method defined by any one of claims 5 to 12, wherein the absorbent matrix contains superabsorbent polymer particles (107, 307) and fluff wood pulp as the hydrophilic fibers.


**Patentansprüche**

1. Körperflüssigkeit absorbierende Matrix (18) mit einem Aggregat von Körperflüssigkeit absorbierendem Material, das zwischen einer flüssigkeitsdurchlässigen ersten Lage (17) auf der der Haut zugekehrten Seite in einem Körperflüssigkeit absorbierenden Kleidungsstück und einer flüssigkeitsdurchlässigen oder flüssigkeitsundurchlässigen zweiten Lage (16) auf einer dem Kleidungsstück zugekehrten Seite gegenüber der der Haut zugekehrten Seite angeordnet ist, wobei:

die absorbierende Matrix hydrophile Fasern umfasst, die als Körperflüssigkeit absorbierendes Material dienen;
die absorbierende Matrix einen Dichtegradienten aufweist, der von der ersten Lage in Richtung der zweiten Lage in einer Dickenrichtung der absorbierenden Matrix zunimmt;
ein Planarorientierungsindex der hydrophilen Fasern in der Dickenrichtung zunimmt;
die absorbierende Matrix (18) eine Oberschichtpartie (22) und eine Unterschichtpartie (21) aufweist, die einander in der Dickenrichtung überlappen, so dass die Oberschichtpartie der ersten Lage und die Unterschichtpartie der zweiten Lage zugekehrt ist;
die Dichte der Oberschichtpartie geringer ist als die der Unterschichtpartie; und
der Planarorientierungsindex in der Oberschichtpartie weniger als 1,30 beträgt und der Planarorientierungsindex in der Unterschichtpartie um mindestens 0,3 höher ist als der Planarorientierungsindex in der Oberschichtpartie.

2. Absorbierende Matrix nach Anspruch 1, wobei die hydrophilen Fasern eine durchschnittliche Faserlänge von höchstens 15 mm haben.

3. Absorbierende Matrix nach Anspruch 1 oder 2, wobei die absorbierende Matrix als hydrophile Fasern mindestens Flockenzellstoff enthält.

4. Absorbierende Matrix nach einem der Ansprüche 1 bis 3, wobei die absorbierende Matrix als hydrophile Fasern hochsaugfähige Polymerteilchen und Flockenzellstoff enthält.

5. Verfahren zur Herstellung einer Körperflüssigkeit absorbierenden Matrix (18) mit einem Aggregat von Körperflüssigkeit absorbierendem Material, das zwischen einer flüssigkeitsdurchlässigen ersten Lage (17) auf der der Haut zugekehrten Seite in einem Körperflüssigkeit absorbierenden Kleidungsstück und einer flüssigkeitsdurchlässigen oder flüssigkeitsundurchlässigen zweiten Lage (16) auf einer dem Kleidungsstück zugekehrten Seite gegenüber der der Haut zugekehrten Seite angeordnet ist, wobei:

das Verfahren mindestens die folgenden Schritte umfasst:

(1) einen Schritt der Bereitstellung der absorbierenden Matrix mit: einer Oberschichtpartie (22) und einer Unterschichtpartie (21), die in der Dickenrichtung aufeinander gestapelt sind und die selbe Dicke haben; und dem Aggregat mit hydrophilen Fasern (105, 305) als Körperflüssigkeit absorbierendes Material, und einer Vielzahl von ersten Aggregaten (501), die jeweils aus einer Partie des zur Bildung der Unterschichtpartien bestimmten Körperflüssigkeit absorbierenden Materials gebildet sind, intermittierend in der Maschinenrichtung angeordnet sind und in der Maschinenrichtung gefördert werden;

(2) einen Schritt des Anordnens der jeweiligen ersten Aggregate (501) zwischen einem Paar von luftdurchlässigen ersten Stützmitteln (201, 202), die in der Maschinenrichtung zwischen einer ersten Düsengruppe (203) für Hochdruck-Dampfstrahl und einem ersten Saugkanal (204) zum Saugen von Dampf verlaufen, so dass die jeweiligen ersten Aggregate unter der Saugwirkung des ersten Saugkanals dem Hochdruck-Dampfstrahl aus der ersten Düsengruppe ausgesetzt werden, um zweite Aggregate (502) zu erhalten, die den in der Dickenrichtung komprimierten ersten Aggregaten entsprechen;

(3) einen Schritt des Stapelns einer Vielzahl von dritten Aggregaten (503) von Körperflüssigkeit absorbierendem Material, das zur Bildung der Oberschichtpartien an den jeweiligen zweiten Aggregaten zur Beschaffung von vierten Aggregaten (504) bestimmt ist; und

(4) einen Schritt des Anordnens der vierten Aggregate zwischen einem Paar von luftdurchlässigen zweiten Stützmitteln (401, 402), die in der Maschinenrichtung zwischen einer zweiten Düsengruppe (403) für Hochdruck-Dampfstrahl und einem zweiten Saugkanal (404) zum Saugen von Dampf verlaufen, die einander zugekehrt sind, so dass die dritten Aggregate in den vierten Aggregaten unter der Saugwirkung des zweiten Saugkanals dem Hochdruck-Dampfstrahl aus der zweiten Düsengruppe ausgesetzt werden, um die vierten Aggregate zu komprimieren und dadurch fünfte Aggregate aus den vierten Aggregaten (505) zu erhalten.

6. Verfahren nach Anspruch 5, wobei das vierte Aggregat einschließlich des auf einer luftdurchlässigen ersten Lage (116) liegenden zweiten Aggregats und einschließlich des mit einer luftdurchlässigen zweiten Lage (117) bedeckten dritten Aggregats zwischen einem Paar von zweiten Stützmitteln angeordnet wird.

7. Verfahren nach Anspruch 5, wobei das auf einer luftdurchlässigen ersten Lage (116) liegende erste Aggregat zusammen mit der ersten Lage zwischen einem Paar von ersten Stützmitteln angeordnet wird.

8. Verfahren nach Anspruch 5, wobei wobei das vierte Aggregat einschließlich des mit einer luftdurchlässigen zweiten Lage (117) bedeckten dritten Aggregats zusammen mit der zweiten Lage zwischen einem Paar von zweiten Stützmitteln angeordnet wird.

9. Verfahren nach einem der Ansprüche 5 bis 8, wobei die ersten Stützmittel und die zweiten Stützmittel Netzförderbänder sind.

10. Verfahren nach einem der Ansprüche 5 bis 9, wobei die hydrophilen Fasern eine Faserlänge von höchstens 15 mm haben.

11. Verfahren nach einem der Ansprüche 5 bis 10, wobei zum Komprimieren des ersten Aggregats anstelle der ersten Düsengruppe und des ersten Saugkanals ein Paar von Presswalzen verwendet wird.

12. Verfahren nach einem der Ansprüche 5 bis 11, wobei wobei die absorbierende Matrix als hydrophile Fasern mindestens Flockenzellstoff enthält.

13. Verfahren nach einem der Ansprüche 5 bis 12, wobei die absorbierende Matrix als hydrophile Fasern hochsaugfähige Polymerteilchen (107, 307) und Flockenzellstoff enthält.

**Revendications**

1. Matrice absorbant les fluides corporels (18) comprenant un agrégat de matériau absorbant les fluides corporels intercalé entre une première feuille perméable aux liquides (17) reposant sur le côté orienté vers la peau dans un article à porter absorbant les fluides corporels et une deuxième feuille perméable aux liquides ou imperméable aux liquides (16) reposant sur un côté orienté vers le vêtement à l'opposé du côté orienté vers la peau, dans laquelle :

la matrice absorbante comprend des fibres hydrophiles servant de matériau absorbant les fluides corporels ;
la matrice absorbante a un gradient de densité qui va en augmentant depuis la première feuille vers le deuxième feuille dans une direction allant dans le sens de l'épaisseur de la matrice absorbante ;
un indice d'orientation plane des fibres hydrophiles augmente dans la direction allant dans le sens de l'épaisseur ;
la matrice absorbante (18) a une partie de couche supérieure (22) et une partie de couche inférieure (21) se chevauchant l'une par rapport à l'autre dans la direction allant dans le sens de l'épaisseur de telle sorte que la partie de couche supérieure est orientée vers la première feuille et la partie de couche inférieure est orientée vers la deuxième feuille ;
la densité de la partie de couche supérieure est inférieure par rapport à celle de la partie de couche inférieure ; et
l'indice d'orientation plane dans la partie de couche supérieure est inférieur à 1,30 et l'indice d'orientation plane dans la partie de couche inférieure fait au moins 0,3 de plus que l'indice d'orientation plane dans la partie de couche supérieure.

2. Matrice absorbante selon la revendication 1, dans laquelle les fibres hydrophiles ont une longueur de fibre moyenne de 15 mm ou moins.

3. Matrice absorbante selon la revendication 1 ou la revendication 2, dans laquelle la matrice absorbante contient au moins de la pâte de bois défibrée pour tenir lieu de fibres hydrophiles.

4. Matrice absorbante selon l'une quelconque des revendications 1 à 3, dans laquelle la matrice absorbante contient des particules de polymère du type à fort pouvoir absorbant et de la pâte de bois défibrée pour tenir lieu de fibres hydrophiles.

5. Procédé permettant de fabriquer une matrice absorbant les fluides corporels (18) comprenant un agrégat de matériau absorbant les fluides corporels intercalé entre une première feuille perméable aux liquides (17) reposant sur le côté orienté vers la peau dans un article à porter absorbant les fluides corporels et une deuxième feuille perméable aux liquides ou imperméable aux liquides (16) reposant sur le côté du côté orienté vers le vêtement à l'opposé du côté orienté vers la peau, dans lequel
le procédé comprend au moins les étapes comme suit :

(1) une étape consistant à mettre en oeuvre la matrice absorbante avec : une partie de couche supérieure (22) et une partie de couche inférieure (21) qui sont empilées l'une sur l'autre dans la direction allant dans le sens de l'épaisseur et qui ont la même épaisseur ; et l'agrégat comprenant des fibres hydrophiles (105, 305) tenant lieu de matériau absorbant les fluides corporels, et une pluralité de premiers agrégats (501) formés, chacun, à partir d'une partie du matériau absorbant les fluides corporels servant à former les parties de couche inférieure sont agencés de manière intermittente dans la direction allant dans le sens machine et sont transportés dans la direction allant dans le sens machine ;
(2) une étape consistant à intercaler les premiers agrégats respectifs (501) entre une paire de premiers moyens de support perméables à l'air (201, 202) s'acheminant dans la direction allant dans le sens machine entre un premier réseau de buses (203) pour jets de vapeur haute pression et un premier orifice d'aspiration (204) à des fins d'aspiration de la vapeur de telle sorte que les premiers agrégats respectifs sont soumis à des jets de vapeur haute pression en provenance du premier réseau de buses sous l'effet d'aspiration du premier orifice d'aspiration pour obtenir des deuxièmes agrégats (502) correspondant aux premiers agrégats comprimés dans la direction allant dans le sens de l'épaisseur de ceux-ci ;
(3) une étape consistant à empiler une pluralité de troisièmes agrégats (503) de matériau absorbant les fluides corporels servant à former les parties de couche supérieure sur les deuxièmes agrégats respectifs pour obtenir des quatrièmes agrégats (504) ; et
(4) une étape consistant à intercaler le quatrième agrégat entre une paire de deuxièmes moyens de support perméables à l'air (401, 402) s'acheminant dans la direction allant dans le sens machine entre un deuxième réseau de buses (403) pour jets de vapeur haute pression et un deuxième orifice d'aspiration (404) à des fins d'aspiration de la vapeur orientés l'un vers l'autre de telle sorte que les troisièmes agrégats dans les quatrièmes agrégats sont soumis à des jets de vapeur haute pression en provenance du deuxième réseau de buses sous l'effet d'aspiration du deuxième orifice d'aspiration pour comprimer les quatrièmes agrégats et pour ainsi obtenir des cinquièmes agrégats à partir des quatrièmes agrégats (505).

**6.** Procédé selon la revendication 5, dans lequel le quatrième agrégat comprenant le deuxième agrégat se trouvant sur une première feuille perméable à l'air (116) et comprenant aussi le troisième agrégat recouvert d'une deuxième feuille perméable à l'air (117) est intercalé entre une paire de deuxièmes moyens de support.

**7.** Procédé selon la revendication 5, dans lequel le premier agrégat se trouvant sur une première feuille perméable à l'air (116) est intercalé avec la première feuille entre une paire de premiers moyens de support.

**8.** Procédé selon la revendication 5, dans lequel le quatrième agrégat comprenant le troisième agrégat recouvert d'une deuxième feuille perméable à l'air (117) est intercalé avec la deuxième feuille entre une paire de deuxièmes moyens de support.

**9.** Procédé selon l'une quelconque des revendications 5 à 8, dans lequel les premiers moyens de support et les deuxièmes moyens de support sont des courroies transporteuses maillées.

**10.** Procédé selon l'une quelconque des revendications 5 à 9, dans lequel les fibres hydrophiles ont une longueur de fibre de 15 mm ou moins.

**11.** Procédé selon l'une quelconque des revendications 5 à 10, dans lequel, au lieu du premier réseau de buses et du premier orifice d'aspiration, une paire de rouleaux de presse est utilisée pour comprimer le premier agrégat.

**12.** Procédé selon l'une quelconque des revendications 5 à 11, dans lequel la matrice absorbante contient au moins de la pâte de bois défibrée pour tenir lieu de fibres hydrophiles.

**13.** Procédé selon l'une quelconque des revendications 5 à 12, dans lequel la matrice absorbante contient des particules de polymère du type à fort pouvoir absorbant (107, 307) et de la pâte de bois défibrée pour tenir lieu de fibres hydrophiles.

[Fig. 1]

[Fig. 2]

[Fig. 3]

[Fig. 4]

[Fig. 5]

(1)XY cross-section(cross-section in MD direction)

XY cross-section for observation

test piece for observation

movement direction
of absorbent matrix

machine direction MD

thickness direction

cross-direction CD

(2)YZ cross-section(cross-section in CD direction)

YZ cross-section for observation

test piece for observation

movement direction
of absorbent matrix

machine direction MD

thickness direction

cross-direction CD

(3)XZ cross-section(cross-section in horizontal direction)

XZ cross-section for observation

test piece for observation

movement direction
of absorbent matrix

machine direction MD

thickness direction

cross-direction CD

[Fig. 6]

[Fig. 7]

[Fig. 8]

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H03173562 A
- JP H10309298 A
- JP H0559602 A
- WO 2011122055 A1
- WO 02056812 A1
- US 2004122394 A1
- JP 2011115547 A